(19) Europäisches Patentamt
European Patent Office
Office européen des brevets

(11) EP 3 374 364 B1

(12) FASCICULE DE BREVET EUROPEEN

(45) Date de publication et mention de la délivrance du brevet:
**01.04.2020 Bulletin 2020/14**

(21) Numéro de dépôt: **16795279.5**

(22) Date de dépôt: **08.11.2016**

(51) Int Cl.:
*C07F 5/02* (2006.01) *C07C 29/14* (2006.01)
*C07C 29/153* (2006.01) *C07C 29/159* (2006.01)
*C07C 31/04* (2006.01) *C07C 209/00* (2006.01)
*C07C 47/04* (2006.01) *C07C 29/147* (2006.01)
*C07C 209/50* (2006.01) *C07C 209/52* (2006.01)
*C07C 45/41* (2006.01)

(86) Numéro de dépôt international:
**PCT/EP2016/077007**

(87) Numéro de publication internationale:
**WO 2017/081022 (18.05.2017 Gazette 2017/20)**

# (54) UTILISATION DE FORMIATES DE BORE POUR LA REDUCTION DE FONCTIONS ORGANIQUES INSATUREES

VERWENDUNG VON BORFORMATEN ZUR REDUKTION UNGESÄTTIGTER ORGANISCHER FUNKTIONEN

USE OF BORON FORMATES FOR REDUCING UNSATURATED ORGANIC FUNCTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.11.2015 FR 1560752**

(43) Date de publication de la demande:
**19.09.2018 Bulletin 2018/38**

(73) Titulaire: **Commissariat à l'Énergie Atomique et aux Énergies Alternatives**
**75015 Paris (FR)**

(72) Inventeurs:
- **CHAUVIER, Clément**
  **75015 Paris (FR)**
- **CANTAT, Thibault**
  **92130 Issy Les Moulineaux (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-2011/045605**

- **ILONA PEUSER ET AL: "CO2 and Formate Complexes of Phosphine/Borane Frustrated Lewis Pairs", CHEMISTRY - A EUROPEAN JOURNAL., vol. 17, no. 35, 22 août 2011 (2011-08-22) , pages 9640-9650, XP055265717, WEINHEIM, DE ISSN: 0947-6539, DOI: 10.1002/chem.201100286**
- **SAMANTHA C. BINDING ET AL: "Heterolytic activation of hydrogen using frustrated Lewis pairs containing tris(2,2',2''-perfluorobiphenyl)borane", DALTON TRANSACTIONS: THE INTERNATIONAL JOURNAL FOR INORGANIC, ORGANOMETALLIC AND BIOINORGANIC CHEMISTRY, vol. 41, no. 30, 1 janvier 2012 (2012-01-01), page 9061, XP055265738, GB ISSN: 1477-9226, DOI: 10.1039/c2dt30334e**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 374 364 B1

## Description

La présente invention concerne un procédé de réduction de composés organiques insaturés choisis dans le groupe formé par les aldéhydes, les cétones, les imines, les acides carboxyliques, les amides, et les esters avec un formiate de bore de formule (I) en présence d'un solvant et éventuellement d'une base.

L'invention concerne également l'utilisation du procédé de réduction de composés organiques insaturés choisis dans le groupe formé par les aldéhydes, les cétones, les imines, les acides carboxyliques, les amides, et les esters selon l'invention, dans la préparation du méthanol, d'amines méthylées, de formaldéhyde et d'alcools ; pour la préparation de réactifs pour les réactions de couplage Suzuki ; et dans la fabrication de vitamines, de produits pharmaceutiques, de colles, de fibres acryliques, de cuirs synthétiques, de pesticides.

Les réactions chimiques de réduction, c'est-à-dire des réactions qui diminuent le nombre d'oxydation formel d'un atome par transfert d'électrons, sont au cœur de la chimie moléculaire contemporaine. En chimie organique, ces réductions sont le plus souvent des transferts d'hydrures ($H^-$, 2 $e^-$ + 2 $H^+$) délivrés à partir de réactifs basés sur des éléments du groupe principal tels que le bore ou l'aluminium ($NaBH_4$, $B_2H_6$, $LiAlH_4$, par exemple) Tous ces réducteurs sont obtenus par l'intermédiaire de procédés coûteux en énergie, le plus souvent à haute température à partir d'hydrures alcalins tels que NaH, LiH ou KH. Ces derniers étant produits industriellement par réaction entre les métaux alcalins fondus et de l'hydrogène moléculaire $H_2$ à hautes températures et pressions. La Figure 1 détaille le procédé industriel de préparation du tétraborohydrure de sodium $NaBH_4$ (TBHS) à partir du borax, source naturelle de l'élément bore.

Le TBHS obtenu de cette manière est alors engagé dans des réactions de réduction notamment de fonctions organiques telles que les carbonyles (aldéhydes et cétones) pour donner des alcools après hydrolyse acide. Il est à noter que les acides carboxyliques sont beaucoup plus difficiles à réduire avec le TBHS. D'autres hydrures comme les hydrures d'aluminium ($LiAlH_4$ par exemple) ou $BH_3$ peuvent être utilisés pour réduire les fonctions organiques telles que les carboxyles y compris les ester, en alcools après une hydrolyse acide.

Néanmoins, il apparait que l'étape de formation des hydrures fait passer le bore d'un état hautement oxydé $B(OCH_3)_3$ à un état fortement réduit $NaBH_4$, ce qui induit nécessairement une grande perte énergétique à cause de l'utilisation d'hydrures alcalins hauts en énergie.

Ainsi, l'utilisation de réducteurs puissants, tels que les hydrures métalliques (hydrure d'aluminium, borohydrure, etc.) conduisent à des réactions fortement exothermiques et ne permettent pas d'assurer un bilan énergétique favorable lors de la réduction de composés organiques présentant une fonction insaturée réductible tels que les aldéhydes, les cétones, les imines, les acides carboxyliques, les amides, les esters, etc.

Certains chercheurs ont rapporté le clivage hétérolytique de l'hydrogène ($H_2$) par une paire de Lewis Frustrée (Frustrated Les Pair en anglais ou FLP), et l'hydrogénation du $CO_2$ avec l'hydrogène obtenu par ledit clivage hétérolytique pour conduire à des formiates de bore comme illustré en Figure 8. En fait, lorsque une FLP réagit avec $H_2$, une espèce de type ($BaseH^+$, $AcideH^-$) se forme et c'est précisément cette espèce qui réagit avec le $CO_2$ pour conduire au formiate de bore ($BaseH^+$, $AcideOCHO^-$). Les formiates de bore anioniques ainsi obtenus ne sont pas capables d'activer l'hydrogène ($H_2$) et ne sont donc pas des espèces réductrices. En fait, une analyse de la littérature fait plutôt apparaître que les formiates de bore ne sont que des agents de transfert du groupe formyle [Organometallics 2013, 32, 2459-2462 ; Tableau 2 page 2461].

Une alternative aux hydrures métalliques précités peut être les systèmes réducteurs qui permettent l'hydrogénation par transfert de composés organiques présentant une fonction insaturée réductible, avec l'acide formique comme donneur exclusif d'hydrogène. En effet, de nombreux autres donneurs sont documentés dans la littérature, notamment l'ester de Hantzch, le cyclohexadiène ou encore l'isopropanol mais, contrairement à l'acide formique, ces derniers ne sont pas obtenus par des procédés économes en énergie et ne réintègrent pas le $CO_2$ dans le cycle énergétique.

L'hydrogénation par transfert est une technique d'hydrogénation dans laquelle la source en hydrogène n'est pas le dihydrogène, mais un autre « donneur en hydrogène ». En synthèse organique, l'hydrogénation par transfert est utile pour la réduction de composés insaturés polaires comme, par exemple, les cétones, les aldéhydes ou les imines.

Les principaux avantages de l'hydrogénation par transfert au moyen de l'acide formique par rapport à l'hydrogénation classique (addition d'une molécule de $H_2$) sont : (i) les réactions ne s'effectuent pas sous haute pression du gaz inflammable $H_2$ et ne demandent ainsi pas d'équipement particulier ; (ii) les donneurs d'hydrogène sont généralement facilement accessibles, peu chers et faciles à manipuler ; (iii) la sélectivité est meilleure et les conditions plus douces et (iv) les sous-produits de la réaction peuvent le plus souvent être recyclés.

La première publication majeure dans le domaine de l'hydrogénation par transfert à l'acide formique date de 1996. Noyori *et coll.* ont en effet rapporté que des cétones pro-chirales telles que l'acétophénone peuvent être réduites stéréosélectivement en utilisant un catalyseur chiral de ruthénium en présence d'acide formique et de triéthylamine [A. Fujii, S. Hashiguchi, N. Uematsu, T. Ikariya, R. Noyori, J. Am. Chem. Soc. 1996, 118, 2521-2522]. Les excès énantiomériques obtenus sont excellents et les rendements le plus souvent supérieurs à 90%. D'un point de vue mécanistique, le formiate de ruthénium [Ru]-OCHO conduit, avec l'assistance du ligand, à l'hydrure de ruthénium [Ru]-H qui permet d'effectuer la réduction du carbonyle de manière concertée. Ces travaux ont mené à l'explosion des recherches sur

l'hydrogénation par transfert comme alternative à l'hydrogénation catalytique, et de nombreux systèmes catalytiques dérivés de celui de Noyori ont été mis au point pour la réduction des carbonyles et des imines [G. Brieger, T. J. Nestrick, Chem. Rev. 1974, 74, 567-580 ; D. Wang, D. Astruc, Chem. Rev. 2015, 115, 6621-6686].

Outre les carbonyles et les imines, les nitro-aromatiques tels que le nitrobenzène $PhNO_2$ peuvent être hydrogénés en présence d'acide formique. Plus précisément, Beller *et coll.* ont rapporté que le complexe $Fe(BF_4)_2{\bullet}6H_2O$ en présence du ligand « tétraphos » $P(CH_2CH_2PPh_2)_3$ dans l'éthanol à 40°C permet d'obtenir les anilines correspondantes avec des rendements dépassant les 77% [G. Wienhofer, I. Sorribes, A. Boddien, F. Westerhaus, K. Junge, H. Junge, R. Llusar, M. Beller, J. Am. Chem. Soc. 2011, 133, 12875-12879]. Il est à noter que la réduction s'effectue sans base, ce qui est rare pour des réductions de ce type, mais dans un large excès d'acide formique (4,5 équivalents d'acide formique par rapport au substrat sont nécessaires). Le même groupe a également démontré que les nitriles aromatiques peuvent être réduits en amine primaire en utilisant du palladium sur charbon Pd/C comme catalyseur en présence de formiate de triethylammonium ($HCOO^-$, $HNEt_3^+$) dans le THF à 40°C. Les rendements obtenus vont de 52 à 98% et seuls les nitriles aromatiques peuvent être réduits : les nitriles aliphatiques restent intacts dans les conditions précédentes.

Outre les fonctions organiques polarisées évoquées précédemment, les alcynes et les alcènes peuvent également être hydrogénés par l'acide formique. Un exemple a été rapporté en 2011 par le groupe de Yin, Han *et coll.* qui ont montré que la réaction entre du Pd(0) ($Pd(PEt_3)_4$), un alcyne et un acide carboxylique conduit au produit d'hydropalladation [R. Shen, T. Chen, Y. Zhao, R. Qiu, Y. Zhou, S. Yin, X. Wang, M. Goto, L. B. Han, J. Am. Chem. Soc. 2011, 133, 17037-17044]. Cette observation les a conduits à établir un protocole de réduction des alcynes en alcènes ou alcanes correspondant. En utilisant le précurseur de palladium $Pd_2(dba)_3$ (dba = dibenzylidèneacétone) avec le ligand dppb (dppb = diphénylphosphinobutane) en présence d'acide formique dans le dioxane à 80°C, un grand nombre d'alcynes (terminaux ou non-terminaux) sont convertis sélectivement en alcène de configuration (Z) exclusivement avec de bons rendements. Lorsque le dppb est remplacé par la tricyclohexylphosphine $PCy_3$, la réduction s'effectue jusqu'à obtention de l'alcane.

En 2008, Elsevier *et coll.* avait déjà montré qu'un complexe de Pd(0) basé sur le ligand carbénique N-hétérocyclique IMes (IMes = 1,3-bis(2,4,6-trimethylphenyl)imidazol-2-ylidene) permettait de réduire des alcynes en alcènes (Z) avec de bonnes sélectivités en présence d'un mélange d'acide formique et de triéthylamine dans le THF ou l'acétonitrile [P. Hauwert, G. Maestri, J. W. Sprengers, M. Catellani, C. J. Elsevier, Angew. Chem. Int. Ed. Engl. 2008, 47, 3223-3226].

Outre la réduction de fonctions organiques insaturées, l'hydrogénation par transfert peut s'effectuer directement sur le donneur d'hydrure à savoir l'acide formique pour obtenir du méthanol, par exemple. Ce type de réaction est appelé réaction de dismutation et est un cas particulier d'hydrogénation par transfert lorsque le donneur présente lui aussi une fonction réductible.

Les premiers travaux dans le domaine ont été réalisés en 2013 par Miller, Goldberg *et coll.* Les auteurs ont montré que le complexe d'iridium $[Cp^*Ir(bpy)(H_2O)](OTf)_2$ (Cp* = pentamethylcyclopentadienyl, bpy = 2,2'-bipyridine) permet d'obtenir des rendements en méthanol allant jusqu'à 12 % [A. J. Miller, D. M. Heinekey, J. M. Mayer, K. I. Goldberg, Angew. Chem. Int. Ed. Engl. 2013, 52, 3981-3984]. Ce chiffre représente la sélectivité de la réaction pour la formation de méthanol comme montré en Figure 2 selon l'équation (3). Cela signifie encore que 88 % des liaisons C-H dans H-COOH se décomposent en dihydrogène selon l'équation (4) de la Figure 3 et non en méthanol. Ces 12 % de sélectivité sont obtenus à 60°C en présence d'acide formique en solution aqueuse (C = 12 M). La conversion de l'acide formique dans ce cas n'est que de 3%.

Plus récemment, Cantat *et al.* ont montré qu'en utilisant un complexe de ruthénium $[Ru(COD)(methylallyl)_2]$ (COD = cyclooctadiène) combiné avec le ligand tridente "triphos" $CH_3C(CH_2PPh_2)_3$ dans le THF à 150°C, la sélectivité (qui correspond ici au rendement en méthanol) peut atteindre 26,7 %. Dans les mêmes conditions et en présence d'un additif acide (*MSA,* acide méthane sulfonique), le rendement dépasse les 50%. Ce dernier résultat représente à l'heure actuelle le rendement le plus important en méthanol obtenu par dismutation de l'acide formique [S. Savourey, G. Lefevre, J. C. Berthet, P. Thuery, C. Genre, T. Cantat, Angew. Chem. Int. Ed. Engl. 2014, 53, 10466-10470].

En dernier lieu, Parkin *et al.* ont démontré en 2015 que des composés de molybdène sont capables d'effectuer la réaction de dismutation de l'acide formique [M. C. Neary, G. Parkin, Chem. Sci. 2015, 6, 1859-1865]. Plus précisément, le complexe $CpMo(CO)_3H$ (Cp = cyclopentadienyle) est le plus efficace des complexes évalués et une sélectivité de 21 % est reportée à 100°C dans le benzène.

La découverte de cette nouvelle réaction a par la suite permis de développer une nouvelle méthodologie de synthèse de méthylamines par une séquence formylation/hydrogénation par transfert [S. Savourey, G. Lefevre, J. C. Berthet, T. Cantat, Chem. Commun. 2014, 50, 14033-14036]. Le système catalytique utilisé dans ce cas est le même que celui developpé par Cantat *et coll.* pour la dismutation de l'acide formique et la réaction est limitée à des anilines primaires et secondaires.

Cet état de l'art révèle que les systèmes réducteurs connus à l'heure actuelle pour promouvoir

- la réduction des composés organiques comportant une fonction insaturée réductible tels que les aldéhydes, les cétones, les imines, les acides carboxyliques, les amides, les esters en présence de l'acide formique, ou
- la réaction de dismutation de l'acide formique en méthanol par exemple,

reposent sur des systèmes métalliques, le plus souvent basés sur des métaux de transition ou des métaux nobles dont le coût, l'abondance et la toxicité posent le plus souvent problème. Il n'existe pas à l'heure actuelle de système non-métallique pouvant effectuer ces transformations avec l'acide formique comme donneur d'hydrure.

ILONA PEUSER ET AL: "CO2 and Formate Complexes of Phosphine/Borane Frustrated Lewis Pairs",CHEMISTRY - A EUROPEAN JOURNAL., vol. 17, no. 35, 22 août 2011 (2011-08-22), pages 9640-9650 divulgue que les FLPs, par exemple composés (13) comprenant le cation [tBu3PH]+, peuvent être utilisés pour la réduction d'oléfines et d'autres substrats organiques et particulièrement l'hydrogénation de CO2. WO2011045605 A1 concerne un procédé comprend le clivage hétérolytique d'hydrogène par une paire de Lewis frustrée comprenant un acide de Lewis et une base de Lewis ; et l'hydrogénation de CO2 avec l'hydrogène clivé hétérolytiquement pour former du méthanol. DALTON TRANSACTIONS: THE INTERNATIONAL JOURNAL FOR INORGANIC, ORGANOMETALLIC AND BIOINORGANIC CHEMISTRY, vol. 41, no. 30, 2012, page 9061 concerne l'activation hétérolytique de l'hydrogène à l'aide de paires de Lewis frustrées contenant du tris (2,2',2"-perfluorobiphényl) borane.

Il existe donc un réel besoin d'un composé non-métallique comme source d'hydrure mettant en jeu l'acide formique et permettant la réduction de composés organiques insaturés réductibles tels que les aldéhydes, les cétones, les imines, les acides carboxyliques, les amides, les esters.

Il existe également un réel besoin d'un composé non-métallique telle que décrit ci-dessus mettant en jeu l'acide formique à la fois comme source d'hydrure et comme substrat à réduire (dismutation) en méthanol, en formaldéhyde ou en amine méthylée par exemple.

En particulier, il existe un réel besoin d'un composé non métallique telle que décrit ci-dessus qui ne contient pas :

- de métaux alcalinoterreux du Groupe IIA du Tableau Périodique des Eléments (comme le magnésium et le calcium) ;
- de métaux de transition du Groupe IB à VIIIB du Tableau Périodique des Eléments (comme le nickel, le fer, le cobalt, le zinc, le cuivre, le rhodium, le ruthénium, le platine, le palladium, l'iridium) ;
- de terres rares dont le numéro atomique est compris entre 57 et 71 (comme le lanthane, le cérium, le praséodyme, le néodyme) ; ou
- d'actinides dont le numéro atomique est compris entre 89 et 103 (comme le thorium, l'uranium).

Il existe en outre un réel besoin d'une source d'hydrure mettant en jeu l'acide formique et permettant la réduction de composés organiques insaturés tels que les aldéhydes, les cétones, les imines, les acides carboxyliques, les amides, et les esters, ladite source

- ayant un contenu énergétique moins élevé que les hydrures métalliques classiques pour effectuer la réduction des composés organiques insaturés indiqués ci-dessus, et/ou
- étant peu coûteuse, et/ou
- étant peu toxique, et/ou
- étant plus sûre comparée aux hydrures connus, par exemple en évitant les réactions violentes avec l'eau ($LiAlH_4$ réagit violemment avec l'eau en libérant de l'hydrogène et doit donc être manipuler dans des conditions rigoureusement anhydres).

La présente invention a pour but de répondre à ces besoins en fournissant un procédé de réduction de composés organiques insaturés choisis dans le groupe formé par les aldéhydes, les cétones, les imines, les acides carboxyliques, les amides, et les esters, selon la revendication 1.

La présente description fournit un procédé de réduction de composés organiques insaturés choisis dans le groupe formé par les aldéhydes, les cétones, les imines, les acides carboxyliques, les amides, et les esters, caractérisé en ce que l'on fait réagir ledit composé organique insaturé avec un formiate de bore de formule (I)

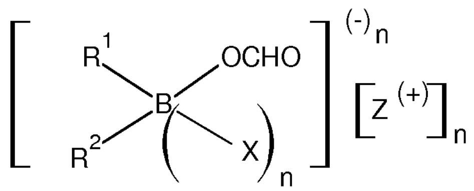

(I)

dans laquelle

- $R^1$ et $R^2$, indépendamment l'un de l'autre, sont choisis dans le groupe formé par un groupe hydroxyle, un groupe alcoxy, un groupe alkyle, un groupe alcényle, un groupe alcynyle, un groupe aryle, un groupe hétéroaryle, un groupe hétérocyclique, un atome d'halogène, un groupe silyle, un groupe siloxy, un groupe phosphino, et un groupe amino, lesdits groupes alkyle, alcényle, alcynyle, alcoxy, silyle, siloxy, aryle, phosphino et amino étant éventuellement substitués ; ou
- $R^1$ et $R^2$ pris ensemble avec l'atome de bore auquel ils sont liés, forment un hétérocycle éventuellement substitué ;
- X est choisi dans le groupe formé par un atome d'hydrogène, un atome d'halogène, un groupe carboxylate, un groupe sulfonate, un groupe hydroxyle, un groupe alcoxy, un groupe alkyle, un groupe alcényle, un groupe alcynyle, un groupe aryle, un groupe hétéroaryle, un groupe hétérocyclique, un groupe silyle, un groupe siloxy, un groupe phosphino,
- Z est un cation choisi dans le groupe formé par une base organique protonnée ayant un pKa supérieur à 3,7 ; $Na^+$ ; $Li^+$ ; $K^+$ ; $Cs^+$ ; le tetraphénylphosphonium ($PPh_4^+$) ; le tetraméthylammonium ($NMe_4^+$) ; le tétraéthylammonium ($NEt_4^+$) ; le tétrabutylammonium ($NBu_4^+$) et le tétraphénylammonium ($NPh_4^+$) ;
- n est un nombre entier choisi parmi 0 ou 1 ;

en présence d'un solvant et éventuellement d'une base organique ou inorganique.

Plus particulièrement, la présente description fournit un procédé de réduction de composés organiques insaturés choisis dans le groupe formé par les aldéhydes, les cétones, les imines, les acides carboxyliques, les amides, et les esters, caractérisé en ce que l'on fait réagir ledit composé organique insaturé avec un formiate de bore de formule (I)

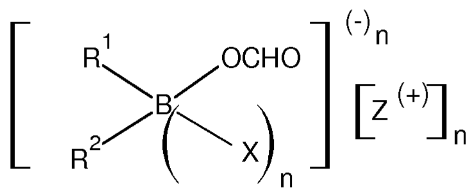

(I)

dans laquelle

- $R^1$ et $R^2$, indépendamment l'un de l'autre, sont choisis dans le groupe formé par un groupe hydroxyle, un groupe alcoxy, un groupe alkyle, un groupe alcényle, un groupe alcynyle, un groupe aryle, un groupe hétéroaryle, un groupe hétérocyclique, un atome d'halogène, un groupe silyle, un groupe siloxy, un groupe phosphino, et un groupe amino, lesdits groupes alkyle, alcényle, alcynyle, alcoxy, silyle, siloxy, aryle, phosphino et amino étant éventuellement substitués ; ou
- $R^1$ et $R^2$ pris ensemble avec l'atome de bore auquel ils sont liés, forment un hétérocycle éventuellement substitué ;
- X est choisi dans le groupe formé par un atome d'halogène, un groupe carboxylate, un groupe sulfonate, un groupe hydroxyle, un groupe alcoxy, un groupe alkyle, un groupe alcényle, un groupe alcynyle, un groupe hétéroaryle, un groupe hétérocyclique, un groupe silyle,
- Z est un cation choisi dans le groupe formé par une base organique protonnée ayant un pKa supérieur à 3,7 choisie dans le groupe formé par le triéthylammonium ($HNEt_3^+$), le di-isopropyléthylammonium ($i$-$Pr_2EtNH^+$), le 2,2,6,6-tétramethylpipéridinium ($TMPH^+$), et le tricyclohexylphosphonium ($HPCy_3^+$) ; $Na^+$ ; $Li^+$ ; $K^+$ ; $Cs^+$ ; le tetraphénylphosphonium ($PPh_4^+$) ; le tetraméthylammonium ($NMe_4^+$) ; le tétraéthylammonium ($NEt_4^+$) ; le tétrabutylammonium ($NBu_4^+$) et le tétraphénylammonium ($NPh_4^+$) ;
- n est un nombre entier égal à 1 ;

en présence d'un solvant et éventuellement d'une base organique ou inorganique.

Ainsi, les formiates de bore de formule (I) peuvent être utilisés pour la réduction de composés organiques présentant une fonction insaturée réductible notamment polaire choisis dans le groupe formé par les aldéhydes, les cétones, les imines, les acides carboxyliques, les amides, et les esters, par transfert d'hydrure.

Le procédé de l'invention permet de réduire les aldéhydes, les cétones, les acides carboxyliques et les esters, en alcools ; les imines en aminés ; et les amides en amines ou en alcools.

Sans vouloir être lié par la théorie, dans le procédé de l'invention, on peut considérer que dans les formiates de bore de formule (I), [B]-OCHO possède des propriétés réductrices et peut être considéré équivalent à [B]-H. Ainsi, la réduction des composés organiques insaturés avec les formiates de bore de formule (I) permet de s'affranchir de

l'utilisation d'hydrure alcalins ou de tout autre métal pour la formation de la liaison B-H qui permet la réduction.

Les formiates de bore de formule (I) dans lesquelles n égal à 1, ne sont pas des FLPs car ce sont des sels classiques qui ne comprennent ni un acide ni une base au sens de Lewis. Pour rappel, une paire de Lewis frustrée est la combinaison d'un acide et d'une base au sens de Lewis dont l'association pour former un adduit de Lewis est empêchée pour des raisons stériques.

L'étape de formation de l'hydrure avec les hydrures alcalins classiques est ainsi supprimée. Dans le procédé de l'invention, l'hydrure est généré, à la demande, *in situ,* à partir du formiate, pour réaliser la réduction des composés organiques insaturés.

Plus précisément, le procédé de l'invention permet de supprimer l'étape de formation de la liaison B-H à partir d'hydrures alcalins, en remplaçant celle-ci par la formation, dans des conditions douces, d'un formiate de bore de formule (I) et d'utiliser ce dernier dans des réactions de réductions de fonctions organiques.

Le procédé d'invention permet de réduire l'impact environnemental négatif des réactions de réduction de la chimie organique utilisant des hydrures de bore générés à partir d'hydrures alcalins en les substituant par des formiates de bore de formule (I). L'un des autres attraits pour une telle technologie repose sur l'utilisation d'acide formique comme équivalent d'hydrure, ce dernier pouvant être obtenu par hydrogénation catalytique du $CO_2$, ce qui a donc pour conséquence de réintégrer le $CO_2$, gaz à effet de serre avéré, dans le cycle énergétique.

La réduction des composés organiques insaturés réductibles précités à l'aide de formiates de bore de formule (I) sans métal selon le procédé de l'invention, évite les inconvénients liés à l'utilisation des métaux, notamment en termes de coût et de toxicité.

Par ailleurs, le procédé de l'invention permet la réduction des composés insaturés précités avec une très bonne sélectivité. Dans certains cas le seul produit obtenu à la suite du procédé de réduction est le produit désiré.

Dans le contexte de l'invention, une paire de Lewis frustrée (Frustrated Lewis Pair en anglais ou FLP) est définie comme une combinaison, au sein du même milieu réactionnel, d'un acide et d'une base au sens de Lewis dont la mutuelle interaction est impossible pour des raisons stériques. Un adduit de Lewis désigne un produit d'une réaction d'addition entre un acide et une base au sens de Lewis, par exemple :

$BH_3 + SMe_2 \rightarrow H_3B{\bullet}SMe_2$
$H_3B{\bullet}SMe_2$ étant ledit adduit de Lewis.

Dans une FLP, la formation des adduits de Lewis n'est pas possible pour des raisons stériques. Par exemple, $B(C_6F_5)_3$ + *t*-$Bu_3P$ ne donne pas $(C_6F_5)_3B{\bullet}P(t\text{-}Bu)_3$. Il n'y a pas d'interaction entre $B(C_6F_5)_3$ qui est l'acide de Lewis et $P(t\text{-}Bu)_3$ qui est la base de Lewis. Une conséquence de cette propriété de « non-interaction mutuelle » consiste à tirer parti, dans un même milieu réactionnel et simultanément, des propriétés acide et basique au sens de Lewis des deux composés. Cette situation a permis l'activation de l'hydrogène moléculaire ($H_2$) « sans metal », autrefois l'apanage des métaux de transitions.

Au sens de la présente invention un groupe « alkyle » désigne un radical carboné linéaire, ramifié ou cyclique, saturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cyclique, on peut citer les radicaux cyclopropyle, cyclobutyle, cyclopentyle, cyclohexyle, cycloheptyle, cyclooctyle, bicylco[2,1,1] hexyle, bicyclo[2,2,1] heptyle. Le groupe alkyle peut comprendre par exemple 1 à 8 atomes de carbone.

Par « alcényle » ou « alcynyle », on entend un radical carboné insaturé linéaire, ramifié ou cyclique, éventuellement substitué, ledit radical carboné insaturé comprenant 2 à 12 atomes de carbone comprenant au moins une double (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés. Comme alcényle cycliques, on peut citer par exemple le cyclopentényle, le cyclohexényle. Les groupes alcényle et alcynyle peuvent comprendre par exemple 2 à 8 atomes de carbone.

Les groupes alkyle, alcényle, alcynyle peuvent être éventuellement substitués par un ou plusieurs groupes hydroxyle ; un ou plusieurs groupes alkoxy ; un ou plusieurs atomes d'halogène choisis parmi les atomes de fluor, chlore, brome ou iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs groupes «siloxy», un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkoxy, alkyle et siloxy tels que définis dans le cadre de la présente invention. Le groupe aryle peut comprendre, par exemple, 6 à 10 atomes de carbone.

Le terme « hétéroaryle » désigne de manière générale un substituant aromatique mono- ou polycyclique comportant de 5 à 12 membres dont au moins 2 atomes de carbone, et au moins un hétéroatome choisi parmi l'azote, l'oxygène, le bore, le silicium, le phosphore ou le soufre. Le groupe hétéroaryle peut être mono- ou poly- cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « hétérocycle ou hétérocyclique » désigne de manière générale un substituant mono- ou polycyclique, comportant de 5 à 10 membres, saturé ou insaturé, contenant de 1 à 4 hétéroatomes choisis indépendamment l'un de l'autre, parmi l'azote, l'oxygène, le bore, le silicium, le phosphore ou le soufre. A titre indicatif, on peut citer le borolane, le borole, le borinane, le 9-borabicyclo[3.3.1]nonane (9-BBN), le 1,3,2-benzodioxaborole (catecholborane ou catBH), pinacholborane (pinBH), les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Le terme « alcoxy » signifie un groupe alkyle, tels que défini ci-dessus, lié par un atome d'oxygène (-O-alkyle).

Par groupe « amino », on entend un groupe de formule $-NR^3R^4$, dans laquelle :

- $R^3$ et $R^4$ représentent, indépendamment l'un de l'autre, un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, silyle, siloxy, tels que définis dans le cadre de la présente invention ; ou
- $R^3$ et $R^4$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

A ce titre, on peut citer, par exemple, le diéthylamino ($-NEt_2$), diphénylamino ($-NPh_2$), le méthyléthylamino (-NMeEt), le bis(triméthylsilyl)amino ($-N(SiCH_3)_2$).

Par groupe « phosphino », on entend un groupe de formule $-PR^5R^6$, dans laquelle :

- $R^5$ et $R^6$ représentent, indépendamment l'un de l'autre, un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, silyle, siloxy, tels que définis dans le cadre de la présente invention ; ou
- $R^5$ et $R^6$, pris ensemble avec l'atome de phosphore auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

A ce titre on peut citer, par exemple, le diéthylphosphino ($-PEt_2$), diphénylphosphino ($-PPh_2$), le méthyléthylphosphino (-PMeEt], le bis(triméthylsilyl)phosphino ($-P(SiCH_3)_2$), le dicyclohexylphosphino ($-PCy_2$).

Par atome d'halogène, on entend un atome choisi parmi les atomes de fluor, chlore, brome et iode.

Le terme sulfonate désigne un groupe de formule $-OSO_2R^7$ dans lequel :

- $R^7$ est choisi parmi : un groupe méthyle ($CH_3$), un groupe trifluorométhyle ($CF_3$), un groupe toluène ($p$-$CH_3C_6H_4$) ou un groupe benzène ($C_6H_5$).

Le groupe « carboxylate » désigne un groupe de formule $-OC(O)R^8$, dans lequel :

- $R^8$ est choisi parmi : un atome d'hydrogène, un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, tels que définis dans le cadre de la présente invention. Plus particulièrement, $R^8$ est choisi parmi un atome d'hydrogène, un groupe alkyle, ou un groupe aryle, lesdits groupes alkyle

ou aryle. A ce titre, on peut citer, par exemple, le formiate (-OC(O)H), l'acétate (-OC(O)$CH_3$), le pivalate (-OC(O)*t*Bu). De préférence, le groupe carboxylate est le formiate (-OC(O)H).

Par groupe « silyle », on entend un groupe de formule [-Si(Y)$_3$] dans lequel chaque Y, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes amino ; un ou plusieurs groupes aryle ; un ou plusieurs groupes siloxy ; avec les groupes alkyle, alkoxy, aryle et siloxy tels que définis dans le cadre de la présente invention. A ce titre on peut citer, par exemple, le triméthylsilyle (TMS), le triéthylsilyle (TES), le *tert*-butyldiphénylsilyle (TBDPS), le *tert*-butyldiméthylsilyle (TBS/TBDMS), le triisopropylsilyle (TIPS), le tri(triméthylsilyl)silyle ou (($CH_3$)$_3$Si)$_3$Si- (TTMS), le tri(*tert*-butyl)silyle ou (($CH_3$)$_3$C)$_3$Si-.

Par groupe « siloxy », on entend un groupe silyle, tel que défini ci-dessus, lié par un atome d'oxygène (-O-Si(Y)$_3$) avec Y tel que défini ci-dessus. A ce titre on peut citer, par exemple, le triméthylsiloxy -OSi($CH_3$)$_3$, le triéthylsiloxy -OSi($CH_2CH_3$)$_3$, le *tert*-butyldiphénylsiloxy -OSi(*t*BuPh$_2$)$_3$.

Dans le contexte de l'invention, la « protonolyse » signifie le clivage d'une liaison chimique par des acides de Brønsted. L'hydrolyse signifie le clivage d'une liaison chimique par l'eau.

Selon un premier mode particulier de réalisation de l'invention, dans le formiate de bore de formule (I),

- $R^1$ et $R^2$, indépendamment l'un de l'autre, sont choisis dans le groupe formé par un groupe alkyle comprenant 1 à 12 atomes de carbone ; un aryle comprenant 6 à 20 atomes de carbone, lesdits groupes alkyle et aryles étant éventuellement substitués.

Dans ce premier mode de réalisation, de préférence, dans le formiate de bore de formule (I),

- $R^1$ et $R^2$, indépendamment l'un de l'autre, sont choisis dans le groupe formé par méthyle, éthyle, propyle, butyle, pentyle, hexyle et leurs isomères ramifiés, cyclohexyle, phényle, benzyle. Encore plus préférentiellement, $R^1$ et $R^2$, indépendamment l'un de l'autre, sont choisis dans le groupe formé par butyle et ses isomères ramifiés, et le cyclohexyle.

Selon un deuxième mode particulier de réalisation de l'invention, dans le formiate de bore de formule (I),

- $R^1$ et $R^2$ pris ensemble avec l'atome de bore auquel ils sont liés, forment un hétérocycle comportant de 5 à 10 membres, ledit hétérocycle étant éventuellement substitué.

Dans ce deuxième mode de réalisation, de préférence, dans le formiate de bore de formule (I),

- $R^1$ et $R^2$ pris ensemble avec l'atome de bore auquel ils sont liés, forment un 9-borabicyclo[3.3.1]nonane (9-BBN), un 1,3,2-benzodioxaborole (catecholborane ou catBH), ou un pinacholborane (pinBH).

Selon un troisième mode particulier de réalisation de l'invention, dans le formiate de bore de formule (I),

- $R^1$ et $R^2$, indépendamment l'un de l'autre, sont choisis dans le groupe formé par un groupe alkyle comprenant 1 à 12 atomes de carbone ; ledit groupe alkyle étant éventuellement substitué.

Dans ce troisième mode de réalisation, de préférence, dans le formiate de bore de formule (I),

- $R^1$ et $R^2$, indépendamment l'un de l'autre, sont choisis dans le groupe formé par méthyle, éthyle, propyle, butyle, pentyle, hexyle et leurs isomères ramifiés, et cyclohexyle. Encore plus préférentiellement, $R^1$ et $R^2$, indépendamment l'un de l'autre, sont choisis dans le groupe formé par butyle et ses isomères ramifiés, et le cyclohexyle.

Dans toutes les variantes et tous les modes de réalisation décrites, dans le formiate de bore de formule (I), X peut avantageusement être choisi dans le groupe formé par

- un atome d'halogène,
- un groupe carboxylate -OCHO,
- un groupe sulfonate de formule -$OSO_2R^7$, dans lequel $R^7$ est choisi parmi un groupe méthyle ($CH_3$) ou un groupe trifluorométhyle ($CF_3$), un groupe toluène (*p*-$CH_3C_6H_4$) ou un groupe benzène ($C_6H_5$),
- un groupe hydroxyle,
- un groupe alcoxy dont le groupe alkyle comprend 1 à 12 atomes de carbone choisis dans le groupe formé par méthyle, éthyle, propyle, butyle, pentyle, hexyle et leurs isomères ramifiés, et

- un groupe alkyle comprenant 1 à 12 atomes de carbone choisis dans le groupe formé par méthyle, éthyle, propyle, butyle, pentyle, hexyle et leurs isomères ramifiés.

Dans toutes les variantes et tous les modes de réalisation décrites, dans le formiate de bore de formule (I), X peut plus avantageusement être choisi dans le groupe formé par un atome d'hydrogène, un atome d'halogène, -OCHO et un groupe sulfonate de formule $-OSO_2R^7$, dans lequel $R^7$ est choisi parmi un groupe méthyle ($CH_3$), un groupe trifluorométhyle ($CF_3$), un groupe toluène ($p$-$CH_3C_6H_4$) ou un groupe benzène ($C_6H_5$).

Dans toutes les variantes et tous les modes de réalisation de l'invention, dans le formiate de bore de formule (I), X est choisi dans le groupe formé par un atome d'halogène, -OCHO et un groupe sulfonate de formule $-OSO_2R^7$, dans lequel $R^7$ est choisi parmi un groupe méthyle ($CH_3$), un groupe trifluorométhyle ($CF_3$), un groupe toluène ($p$-$CH_3C_6H_4$) ou un groupe benzène ($C_6H_5$).

Dans toutes les variantes et tous les modes de réalisation de l'invention, dans le formiate de bore de formule (I), Z est un cation choisi dans le groupe formé par une base protonnée ayant un pKa supérieur à 3,7 choisie dans le groupe formé par le triéthylammonium ($HNEt_3^+$), le di-isopropyléthylammonium ($i$-$Pr_2EtNH^+$), le 2,2,6,6-tétramethylpipéridinium ($TMPH^+$), et le tricyclohexylphosphonium ($HPCy_3^+$) ; $Na^+$ ; $Li^+$ ; $K^+$ ; $Cs^+$ ; le tetraphénylphosphonium ($PPh_4^+$) ; le tetraméthylammonium ($NMe_4^+$) ; le tétraéthylammonium ($NEt_4^+$) ; le tétrabutylammonium ($NBu_4^+$) et le tétraphénylammonium ($NPh_4^+$).

Dans toutes les variantes et tous les modes de réalisation de l'invention, dans le formiate de bore de formule (I), Z est plus préférentiellement un cation choisi dans le groupe formé par le triéthylammonium ($HNEt_3^+$), le di-isopropyléthylammonium ($i$-$Pr_2EtNH^+$), le 2,2,6,6-tétramethylpipéridinium ($TMPH^+$), le tricyclohexylphosphonium ($HPCy_3^+$), et $Na^+$. Encore plus préférentiellement, Z peut être un cation choisi dans le groupe formé par le triéthylammonium ($HNEt_3^+$), le tricyclohexylphosphonium ($HPCy_3^+$), et $Na^+$.

De préférence, dans toutes les variantes et tous les modes de réalisation de l'invention, les formiates de bore de formule (I) sont [$Et_3NH^+$, $BCy_2(OCHO)_2^-$], [$i$-$Pr_2EtNH^+$, $BCy_2(OCHO)_2^-$], [$Et_3NH^+$, $n$-$Bu_2B(OCHO)_2^-$], [$Et_3NH^+$, $BBN(OCHO)_2^-$], [$i$-$Pr_2EtNH^+$, $BBN(OCHO)_2^-$], [$Na^+$, $BBN(OCHO)_2^-$], [$Cy_3PH^+$, $BBN(OCHO)_2^-$] et [$TMPH^+$, $BBN(OCHO)_2^-$]. Plus préférentiellement, les formiates de bore de formule (I) sont [$Et_3NH^+$, $BBN(OCHO)_2^-$], [$i$-$Pr_2EtNH^+$, $BBN(OCHO)_2^-$], [$Na^+$, $BBN(OCHO)_2^-$], [$Cy_3PH^+$, $BBN(OCHO)_2^-$] et [$TMPH^+$, $BBN(OCHO)_2^-$].

Dans un quatrième mode particulier de réalisation décrit, lorsque n=0, le formiate de bore est de formule générale (Ia)

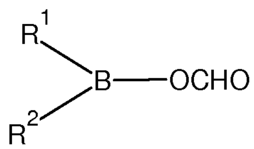

(Ia)

dans laquelle $R^1$ et $R^2$ sont tels que définis précédemment. La réduction des composés organiques insaturés nécessite alors la présence d'une base organique ou inorganique.

Lorsque la base est une base inorganique, il s'agit d'un composé minéral présentant des propriétés basiques au sens de Brønsted. La base inorganique peut être choisie parmi le carbonate de sodium ($Na_2CO_3$), le carbonate de potassium ($K_2CO_3$), l'hydrogénocarbonate de sodium ($NaHCO_3$), l'hydrure de sodium (NaH), ou l'hydrure de potassium (KH).

La base est de préférence organique.

La base organique peut être choisie parmi :

- les bases organiques azotées qui sont avantageusement des amines tertiaires ou secondaires choisies dans le groupe formé par, par exemple, la triéthylamine, la triméthylamine, la N-diisopropyléthylamine (DIPEA), la diéthylisopropylamine (DIEA), la 7-Methyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), la *N*-méthylpiperidine, la 2,2,6,6-tétraméthylpipéridine (TMP), la pipéridine, la N-méthylaniline, la pyrrolidine, la morpholine et la diisopropylamine (DIPA).
- les bases organiques phosphorées pouvant être des alkyle ou aryles phosphines choisies dans le groupe formé par, par exemple, la triphénylphosphine, le 2,2'-bis(diphénylphosphino)-1,1'-binaphthyle (BINAP), le triisopropylphosphine, 1,2-bis(diphénylphosphino)éthane (dppe), la tricyclohexylphosphine ($PCy_3$) ; les aza-phosphines choisies parmi, par exemple, le 2,8,9-triisopropyl-2,5,8,9-tetraaza-1-phosphabicyclo[3.3.3]undecane ($BV^{Me}$) et le 2,8,9-triisobutyl-2,5,8,9-tetraaza-1-phosphabicyclo[3.3.3]undecane ($BV^{iBu}$) ;
- les bases carbonées pour lesquelles la protonation a lieu sur un atome de carbone, choisies avantageusement

parmi les carbènes N-hétérocycliques issus d'un sel d'imidazolium, ledits carbènes étant, par exemple, choisis dans le groupe formé par les sels de 1,3-bis(2,6-diisopropylphenyl)-1H-imidazol-3-ium (également appelé IPr), 1,3-bis(2,6-diisopropylphényl)-4,5-dihydro-1H-imidazol-3-ium (également appelé s-IPr), 1,3-bis(2,4,6-triméthylphényl)-1H-imidazol-3-ium (également appelé IMes), 1,3-bis(2,4,6-triméthylphényl)-4,5-dihydro-1H-imidazol-3-ium (également s-IMes), 4,5-dichloro-1,3-bis(2,6-diisopropylphényl)-1H-imidazol-3-ium (également appelé $Cl_2$-IPr), 1,3-di-tert-butyl-1H-imidazol-3-ium (également appelé ItBu), et 1,3-di-tert-butyl-4,5-dihydro-1H-imidazol-3-ium (également appelé s-ItBu), lesdits sels étant sous la forme de sels de chlorure ou de tétraphenylborate, par exemple.

Des exemples de carbènes N-hétérocycliques sont représentés dans la Figure 4.

De préférence, la base organique est une base organique azotée choisie dans le groupe formé par la triéthylamine, la triméthylamine, la N-diisopropyléthylamine (DIPEA), la diéthylisopropylamine (DIEA), la 7-Methyl-1,5,7-triazabicyclo[4.4.0]dec-5-ène (MTBD), le 1,8-diazabicyclo[5.4.0]undéc-7-ène (DBU), , la 2,2,6,6-tétraméthylpipéridine (TMP) et la diisopropylamine (DIPA).

Dans ce quatrième mode particulier de réalisation décrit ; la quantité de base est de 0,05 à 3 équivalents molaires de préférence de 0,5 à 1,5 équivalent molaire bornes incluses, par rapport au formiate de bore de formule (la).

Dans un cinquième mode particulier de réalisation de l'invention, lorsque n=1, le formiate de bore est de formule générale (Ib)

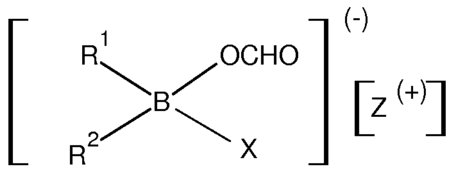

(Ib)

dans laquelle $R^1$, $R^2$, X et Z sont tels que définis précédemment. La réduction des composés organiques insaturés ne nécessite alors pas la présence d'une base.

Comme déjà indiqué, les formiates de bore de formule (Ib) ne sont pas des paires de Lewis frustrées (Frustrated Lewis Pair en anglais ou FLP). Ce sont des sels classiques comprenant un résidu cationique ($Z^+$) et un résidu anionique (le formiate de bore).

Dans tous les modes de réalisation et variante de l'invention, lorsque cela s'avère nécessaire, dans le procédé de réduction selon l'invention un additif peut également être utilisé pour solubiliser les réactifs de départ. Par additif, au sens de l'invention, on entend tout composé capable d'améliorer la solubilité des réactifs intervenant dans le procédé de réduction de l'invention. Ces additifs sont introduits, par exemple, lorsque les sels alcalins de formiate de bore de formule (I) sont employés (par exemple $Na^+$ ou $K^+$). Ces additifs peuvent être choisis, par exemple, parmi :

- les éthers couronnes choisis dans le groupe formé par le 12-couronne-4, le 15-couronne-5, le 18-couronne-6, le dibenzo-18-couronne-6, le benzo-18-couronne-6, le benzo-15-couronne-5, et le dibenzo-15-couronne-5 ;
- les aza-couronnes choisis dans le groupe formé par le 1,4,7,10-tétraazacyclododécane (cyclène), le 1,4,7,10,13,16-hexaazacyclooctadécane (hexacyclène), et le diaza-18-couronne-6 ;
- les thioéthers couronnes choisis dans le groupe formé parle 1,5,9,13-tétrathiacyclohexadécane (16-Ane-$S_4$), et le 1,4,7,10,13,16-hexathiacyclooctadécane (18-Ane-$S_6$).

Les conditions opératoires décrites pour le procédé de réduction des composés organiques insaturés précités par les formiates de formule (I) selon l'invention, s'appliquent à tous les modes de réalisation et variantes de l'invention, y compris les formiates de bore de formule (la) et (Ib).

La quantité des composés organiques insaturés à réduire est de 0,5 à 2 équivalents molaires, de préférence 0,5 à 1,1 équivalents molaires, bornes incluses, par rapport au formiate de bore de formule (I).

Le procédé de réduction par les formiates de formule (I) peut avoir lieu dans un ou un mélange d'au moins deux solvants. Le solvant peut être choisis dans le groupe formé par :

- les éthers choisis parmi l'éther diéthylique, le THF, le diglyme, le 1,4-dioxane ;
- les hydrocarbures choisis parmi le benzène, ou le toluène ;
- les solvants azotés choisis parmi la pyridine, ou l'acétonitrile ;
- les sulfoxydes choisis parmi le diméthylesulfoxyde ;
- les halogénures d'alkyle choisis parmi le chloroforme, ou le chlorure de méthylène ; et

- un fluide supercritique choisis parmi le $CO_2$ supercritique.

Le procédé de réduction est préférentiellement conduit dans un solvant polaire aprotique tel que le THF ou l'acétonitrile et plus préférentiellement dans l'acétonitrile.

Le procédé de réduction de l'invention a lieu à une température supérieure à 25°C, de préférence entre 30 et 200°C, plus préférentiellement entre 50 et 180, encore plus préférentiellement entre 80 et 130°C, bornes incluses.

La durée de la réduction dépendra du type de liaison à réduire et du formiate de bore de formule (I) employé pour effectuer la réduction. En général, la durée du procédé de réduction est de 30 min à 72 h, de préférence de 2 h à 24 h bornes incluses.

Lorsqu'un additif est utilisé, la quantité de l'additif est de 1 à 2 équivalents molaires de préférence de 1 à 1,5 équivalents molaires bornes incluses, par rapport au formiate de bore de formule (I).

Les composés organiques insaturés choisis dans le groupe formé par les aldéhydes, les cétones, les imines, les acides carboxyliques, les amides, et les esters à réduire sont, en général, des composés commerciaux ou peuvent être préparés par tout procédé connu de l'homme du métier.

Le procédé de réduction de l'invention génère une pression de gaz résultant de la formation de dioxyde de carbone et éventuellement de dihydrogène. La réaction peut alors se produire sous pression des gaz formés ou sous pression atmosphérique en collectant les gaz, par exemple dans une burette. Ce(s) gaz peuvent être réutilisés pour préparer l'acide formique ou l'un de ses dérivés par exemple pour effectuer la réduction du $CO_2$ à $2e^-$.

Comme déjà indiqué, le procédé de l'invention permet de réduire les aldéhydes, les cétones, les acides carboxyliques et les esters, en alcools ; les imines en aminés ; et les amides en amines ou en alcools. Ces réactions sont illustrées en Figure 6 avec $R^1$, $R^2$ et $R^3$ comme définis précédemment.

Lorsque dans le formiate de bore de formule (I) ou (Ib) X représente -OCHO ou lorsque le formiate de bore est de formule (la) et que le produit de réduction désiré est le méthanol, le formaldéhyde ou une amine méthylée, l'ajout d'un substrat insaturé n'est pas nécessaire car ces produits peuvent être obtenus dans des conditions de dismutation. La dismutation constitue un sixième mode particulier de réalisation de l'invention. Des exemples de réaction de dismutation sont représentés dans la Figure 7. En effet, dans les conditions de dismutation, le substrat insaturé réductible est le formiate qui est déjà présent dans le formiate de bore de formule (I) ou (Ib) ou (la). Dans le contexte de l'invention, « dismutation » signifie que le substrat à réduire et la source d'hydrure sont le même résidu, à savoir, le formiate -OCHO.

Dans le cas de la dismutation de l'acide formique en une amine méthylée, une amine primaire ou secondaire doit être introduite dans le milieu réactionnel si celle-ci n'est pas déjà incluse dans le formiate de bore de formule (Ib) sous forme protonnée (et dans ce cas précis, $Z^+$ est alors une amine primaire ou secondaire protonnée).Ceci permettra de converger vers l'obtention de l'amine méthylée plutôt que vers le méthoxyborane.

Sans vouloir être par une théorie, dans les conditions de dismutation, l'atome de bore présent dans le formiate de bore de formule (I) ou (Ib) permet le transfert d'hydrure du formiate -OCHO au bore avec formation de B-H, puis le transfert d'hydrure du bore vers le formiate pour effectuer la réduction. Ainsi, une partie du formiate est utilisée pour fournir les hydrures et une partie est utilisée comme substrat pour être réduit.

Dans le cas de la réaction de dismutation, la thermolyse à une température supérieure à 25°C, de préférence entre 30 et 200°C, plus préférentiellement entre 50 et 180, encore plus préférentiellement entre 80 et 130°C, bornes incluses, du formiate de bore de formule (I) ou (Ib) donnera le produit de réduction. L'obtention de méthanol et de formaldehyde nécessite une hydrolyse (ajout d'eau) en fin de réaction suivie d'une distillation et/ou d'un transfert sous vide. Dans le cas des amines méthylées, ces dernières peuvent être récupérées directement par distillation et/ou transfert sous vide du brut réactionnel. Si l'amine méthylée n'est pas un composé volatil, une chromatographie sur colonne permettra de l'isoler sous forme pure.

Les formiates de bore de formule (I), (Ia) et (Ib) peuvent être préparés par un procédé dans lequel on fait réagir un organoborane de formule (II)

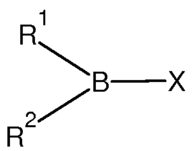

(II)

dans laquelle $R^1$, $R^2$ et X sont tels que définis précédemment, avec l'acide formique, ou avec l'un de ses dérivés de formule $HCO_2Z$ dans laquelle Z est tel que défini ci-dessus, ou avec un mélange d'acide formique et d'au moins un de ses dérivés ;

éventuellement en présence d'une base organique ou inorganique.

L'un des attraits de ce procédé repose sur l'utilisation d'acide formique qui peut être obtenu par hydrogénation

catalytique du $CO_2$, ce qui a pour conséquence de réintégrer le $CO_2$, gaz à effet de serre avéré, dans le cycle énergétique.

Par ailleurs, l'utilisation des formiates de bore de formule (I), (Ia) ou (Ib) peut présenter d'autres avantages, notamment :

- L'acide formique utilisé pour leur préparation est liquide dans les conditions normales de température et pression, ce qui le rend facile à transporter avec des coûts (et donc des risques) plus faibles que le transport de gaz tel que l'hydrogène. Ceci est particulièrement intéressant lorsque la production de l'acide formique et le procédé de préparation des formiates de bore sont effectuées dans deux lieux distincts. Cette étape revient donc à stocker l'hydrogène sous forme d'acide formique.
- L'acide formique est non toxique en solution diluée (concentration inférieure à 85% massique dans l'eau) et est, de fait, un réactif bénin. Ceci est à comparer notamment à l'étape de formation de l'hydrure de sodium présenté dans la Figure 1, ou le sodium fondu et l'hydrogène sont mis en réaction. Les surcoûts liés aux mesures de sécurité drastiques nécessaire à la mise en place de ce genre de procédé sont en effet importants.
- Les organoboranes de formule (II) utilisés pour la préparation des formiates de bore de formule (I), (Ia) ou (Ib) sont commerciaux, ou facilement préparés par l'homme du métier à partir de réactifs commerciaux et bon marché. A contrario, lorsque des métaux sont utilisés, les ligands employés pour les stabiliser sont coûteux et leur préparation nécessite le plus souvent de multiples étapes de synthèse.

Toutes les définitions, modes de réalisation et modes préférentiels décrits précédemment pour les formiates de bore de formule (I), (Ia) ou (Ib) notamment en ce qui concerne les substituants $R^1$, $R^2$, X, s'appliquent également aux organoboranes de formule (II).

Les conditions opératoires décrites pour le procédé de préparation de formiates de formule (I), s'appliquent également à tous les modes de réalisation et variantes de l'invention y compris la préparation des formiates de bore de formule (Ia) et (Ib).

Dans le procédé de préparation des formiates de bore de formule (I), (Ia) ou (Ib), l'organoborane de formule (II) est, de préférence, choisi dans le groupe formé par le dicyclohexylborane, le dicyclohexylborane triflate ($Cy_2BOSO_2CF_3$), le iododicyclohexylborane, le chlorodicyclohexylborane, le dibutylborane, le dibutylborane methanesulfonate (*n*-$Bu_2$BOSO2Me), le 9-borabicyclo[3.3.1]nonane (9-BBN), le B-méthoxy-9-borabicyclo[3.3.1]nonane (B-OMe-9-BBN), le B-iodo-9-borabicyclo[3.3.1]nonane (B-I-9-BBN), B-bromo-9-borabicyclo[3.3.1]nonane (B-Br-9-BBN), le B-benzyl-9-borabicyclo[3.3.1]nonane, le Me-TBD-$BBN^+$I.

Le procédé de préparation de formiates de bore de formule (I), (Ia) ou (Ib) peut avoir lieu en présence d'une base organique ou inorganique.

Lorsque la base est une base inorganique, il s'agit d'un composé minéral présentant des propriétés basiques au sens de Brønsted. La base inorganique peut être choisie parmi le carbonate de sodium ($Na_2CO_3$), le carbonate de potassium ($K_2CO_3$), l'hydrogénocarbonate de sodium ($NaHCO_3$) l'hydrure de sodium (NaH), ou l'hydrure de potassium (KH).

La base est de préférence organique.

La base organique peut être choisie parmi :

- les bases organiques azotées qui sont avantageusement des amines tertiaires ou secondaires choisies dans le groupe formé par, par exemple, la triéthylamine, la triméthylamine, la N-diisopropyléthylamine (DIPEA), la diéthylisopropylamine (DIEA), la 7-Methyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), la *N*-méthylpiperidine, la 2,2,6,6-tétraméthylpipéridine (TMP), la pipéridine, la N-méthylaniline, la pyrrolidine, la morpholine et la diisopropylamine (DIPA).
- les bases organiques phosphorées pouvant être des alkyle ou aryles phosphines choisies dans le groupe formé par, par exemple, la triphénylphosphine, le 2,2'-bis(diphénylphosphino)-1,1'-binaphthyle (BINAP), le triisopropylphosphine, 1,2-bis(diphénylphosphino)éthane (dppe), la tricyclohexylphosphine ($PCy_3$) ; les aza-phosphines choisies parmi, par exemple, le 2,8,9-triisopropyl-2,5,8,9-tetraaza-1-phosphabicyclo[3.3.3]undecane ($BV^{Me}$) et le 2,8,9-triisobutyl-2,5,8,9-tetraaza-1-phosphabicyclo[3.3.3]undecane ($BV^{iBu}$) ;
- les bases carbonées pour lesquelles la protonation a lieu sur un atome de carbone, choisies avantageusement parmi les carbènes N-hétérocycliques issus d'un sel d'imidazolium, ledits carbènes étant, par exemple, choisis dans le groupe formé par les sels de 1,3-bis(2,6-diisopropylphenyl)-1H-imidazol-3-ium (également appelé IPr), 1,3-bis(2,6-diisopropylphényl)-4,5-dihydro-1H-imidazol-3-ium (également appelé s-IPr), 1,3-bis(2,4,6-triméthylphényl)-1H-imidazol-3-ium (également appelé IMes), 1,3-bis(2,4,6-triméthylphényl)-4,5-dihydro-1H-imidazol-3-ium (également s-IMes), 4,5-dichloro-1,3-bis(2,6-diisopropylphényl)-1H-imidazol-3-ium (également appelé $Cl_2$-IPr), 1,3-di-tert-butyl-1H-imidazol-3-ium (également appelé ItBu), et 1,3-di-tert-butyl-4,5-dihydro-1H-imidazol-3-ium (également appelé s-ItBu), lesdits sels étant sous la forme de sels de chlorure ou de tétraphenylborate, par exemple.

Des exemples de carbènes N-hétérocycliques sont représentés dans la Figure 4.

De préférence, la base organique est une base organique azotée choisie dans le groupe formé par la triéthylamine, la triméthylamine, la N-diisopropyléthylamine (DIPEA), la diéthylisopropylamine (DIEA), la 7-Methyl-1,5,7-triazabicyclo[4.4.0]dec-5-ène (MTBD), le 1,8-diazabicyclo[5.4.0]undéc-7-ène (DBU), , la 2,2,6,6-tétraméthylpipéridine (TMP) et la diisopropylamine (DIPA).

Lorsque cela s'avère nécessaire, un additif peut également être utilisé pour solubiliser les réactifs de départ. Par additif, au sens de l'invention, on entend tout composé capable d'améliorer la solubilité des réactifs intervenant dans le procédé de préparation des formiates de bore de formule (I). Ces additifs peuvent être choisis, par exemple, parmi :

- les éthers couronnes choisis dans le groupe formé par le 12-couronne-4 (12-C-4), le 15-couronne-5 (15-C-5), le 18-couronne-6 (18-C-6), le dibenzo-18-couronne-6 (DB18-C-6), le benzo-18-couronne-6 (B18-C-6), le benzo-15-couronne-5 (C15-C-5), et le dibenzo-15-couronne-5 (DB15-C-5) ;
- les aza-couronnes choisis dans le groupe formé par le 1,4,7,10-tétraazacyclododécane (cyclène), le 1,4,7,10,13,16-hexaazacyclooctadécane (hexacyclène), et le diaza-18-couronne-6 ;
- les thioéthers couronnes choisis dans le groupe formé parle 1,5,9,13-tétrathiacyclohexadécane (16-Ane-$S_4$), et le 1,4,7,10,13,16-hexathiacyclooctadécane (18-Ane-$S_6$).

Les organoboranes de formule (II) peuvent, le cas échéant, être immobilisés sur des supports hétérogènes afin d'assurer leur séparation facile et/ou leur recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; l'alumine ; ou le chlorure de magnésium ($MgCl_2$).

L'acide formique ou l'un de ses dérivés comme définis précédemment peut être préparé par tout procédé connu de l'homme du métier. Dans le présent exposé, des procédés connus pour pouvoir réaliser cette étape ont été décrits. De préférence, l'acide formique est préparé par une électro-réduction à 2 $e^-$ ou une hydrogénation catalytique du $CO_2$ comme montré en Figure 5. Les dérivés de l'acide formique sont préparés à partir de celui-ci ou obtenus directement par exemple par hydrogénation du $CO_2$.

L'hydrogénation catalytique du $CO_2$ nécessite l'emploi d'une base pour déplacer l'équilibre vers la formation du sel de formiate. Ainsi, lorsque l'hydrogénation catalytique du $CO_2$ est choisie et que dans le procédé de l'invention une base est utilisée, les deux réactions peuvent être réalisées à la suite dans le même réacteur.

Si l'électro-réduction à 2$e^-$ du $CO_2$ est choisie pour préparer l'acide formique et que dans le procédé de préparation de formiates de bore de formule (I) une base est utilisée, les deux réactions peuvent être réalisées à la suite dans le même réacteur. La base devra alors être ajoutée.

La réduction à 2$e^-$ du $CO_2$ est préférentiellement effectuée selon les méthodes les plus sélectives connues à ce jour. Par exemple, l'hydrogénation catalytique du $CO_2$ peut être effectuée selon le protocole de Nozaki *et coll.* [R. Tanaka, M. Yamashita, K. Nozaki, J. Am. Chem. Soc. 2009, 131, 14168-14169] et permet de récupérer du formiate de potassium. L'éléctro-réduction peut quant à elle être conduite selon les conditions de Shibata et coll. ou de Hori *et coll.* [Y. Hori, H. Wakebe, T. Tsukamoto, O. Koga, Electrochim. Acta 1994, 39, 1833-1839 ; N. Furuya, T. Yamazaki, M. Shibata, J. Electroanal. Chem. 1997, 431, 39-41]

Il est à noter que les électro-réductions sont dépendantes d'un grand nombre de paramètres tels que la nature du réacteur, les électrodes, l'électrolyte ou encore l'électro-catalyseur utilisé. Ces notions et les différents systèmes permettant d'obtenir de l'acide formique ou un de ses dérivés sont discutés dans les revues d'Olah et coll., de Leung, Xuan et coll. ou encore de Kenis et coll. [A. Goeppert, M. Czaun, J. P. Jones, G. K. Surya Prakash, G. A. Olah, Chem. Soc. Rev. 2014, 43, 7995-8048 ; H.-R. M. Jhong, S. Ma, P. J. A. Kenis, Curr. Opin. Chem. Eng. 2013, 2, 191-199 ; X. Lu, D. Y. C. Leung, H. Wang, M. K. H. Leung, J. Xuan, ChemElectroChem 2014, 1, 836-849]. L'acide formique ou l'un de ses dérivés (formiate de sodium, de potassium, de lithium, de césium, d'ammonium, par exemple) ainsi formé peut ensuite être engagé dans l'étape de réduction.

Les différents réactifs utilisés dans le procédé de préparation des formiates de formule (I) (*i.e.* les organoboranes de formule (II), l'acide formique ou l'un de ses dérivés, la base, l'additif) sont, en général, des composés commerciaux ou peuvent être préparés par tout procédé connu de l'homme du métier.

La quantité de l'organoborane de formule (II) est de 0,05 à 1 équivalent molaire, de préférence de 0,5 à 1 équivalent molaire, bornes incluses, par rapport à l'acide formique ou à son(ses) dérivé(s), ou à un mélange d'acide formique et d'au moins un de ses dérivés.

Lorsqu'une base est utilisée, la quantité de base est de 0,05 à 3 équivalents molaires de préférence de 0,5 à 1,5 équivalent molaire bornes incluses, par rapport à l'acide formique ou à son(ses) dérivé(s), ou à un mélange d'acide formique et d'au moins un de ses dérivés.

Lorsqu'un additif est utilisé, la quantité de l'additif est de 1 à 2 équivalents molaires de préférence de 1 à 1,5 équivalents molaires bornes incluses, par rapport au(x) dérivé(s) de l'acide formique ou à un mélange d'acide formique et d'au moins un de ses dérivés.

Le nombre d'équivalents de l'organoborane de formule (II), de la base et de l'additif sont en fait calculés par rapport au nombre d'équivalents de $HCOO^-$. Par exemple, dans le cas d'un mélange d'acide formique avec au moins un de ses dérivés comme par exemple, un mélange HCOOH/HCOONa (1:1), le nombre d'équivalents formels de $HCOO^-$ est de 2.

Le procédé de préparation des formiates de bore de formule (I), (Ia) ou (Ib) peut avoir lieu à une température comprise entre -78°C et 150°C, par exemple entre 0 et 100°C, par exemple entre 0 et 30°C, par exemple entre 15 et 25°C, bornes incluses.

La durée de la réaction dépend du taux de conversion de l'acide formique. La réaction peut être effectuée pendant une durée de 5 minutes à 200 heures, de préférence de 10 minutes à 24 heures, bornes incluses.

Le procédé de préparation de formiates de bore de formule (I), (Ia) ou (Ib) peut être effectuée dans l'acide formique pur, dans l'un des dérivés de l'acide formique si celui-ce est liquide ou en utilisant un ou un mélange d'au moins deux solvants. Le solvant peut être choisi dans le groupe formé par :

- les éthers, de préférence, l'éther diéthylique, le THF, le diglyme, le 1,4-dioxane ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène ; et
- un fluide supercritique, de préférence ; le $CO_2$ supercritique.

Le procédé de préparation du formiate de bore est préférentiellement conduit dans un solvant aprotique tel que le THF, le benzène ou le toluène.

L'invention a également pour objet, l'utilisation du procédé de réduction de composés organiques insaturés choisis dans le groupe formé par les aldéhydes, les cétones, les imines, les acides carboxyliques, les amides, et les esters avec les formiates du bore de formule (I) selon l'invention,

- pour la préparation du méthanol, de amines méthylées de formule $R^1R^2N-CH_3$, de formaldéhyde et d'alcools de formule $R^1CH_2OH$ avec $R^1$ et $R^2$ tels que définis précédemment ;
- pour la préparation de réactifs pour les réactions de couplage Suzuki,
- dans la fabrication de vitamines, de produits pharmaceutiques, de colles, de fibres acryliques, de cuirs synthétiques, de pesticides.

Un autre objet de l'invention concerne un procédé de préparation de méthanol comprenant (i) une étape de réduction de l'acide formique ou de l'un de ses esters de formule $HCO_2R^8$ dans laquelle $R^8$ est tel que défini ci-dessus, avec un formiate de bore de formule (I) selon le procédé de l'invention, et éventuellement (ii) une étape d'hydrolyse. A l'issue de l'hydrolyse, éventuellement une distillation ou une concentration sous vide peut s'avérer nécessaire.

L'invention a également pour objet, un procédé de préparation d'amines méthylées de formule $R^1R^2N-CH_3$ avec $R^1$ et $R^2$ tels que définis précédemment, comprenant (i) une étape de réduction de l'acide formique en présence d'une amine primaire ou d'une amine secondaire, avec un formiate de bore de formule (I) selon le procédé de l'invention, et (ii) une distillation ou une concentration sous vide ou une chromatographie sur colonne.

L'invention a également pour objet, un procédé de préparation de formaldéhyde, comprenant une étape de réduction de l'acide formique ou de l'un de ses esters de formule $HCO_2R^8$ dans laquelle $R^8$ est tel que défini ci-dessus, avec un formiate de bore de formule (I) selon le procédé de l'invention, et éventuellement (ii) une étape d'hydrolyse. Après hydrolyse, éventuellement une distillation ou une concentration sous vide pourra s'avérer nécessaire.

L'invention a également pour objet, un procédé de préparation d'un alcool de formule $R^1CH_2OH$ avec $R^1$ tel que défini précédemment, comprenant (i) une étape de réduction d'un composé organique insaturé choisi dans le groupe formé par les aldéhydes, les acides carboxyliques et les esters avec un formiate de bore de formule (I) selon le procédé de l'invention, et éventuellement (ii) une étape d'hydrolyse. Après hydrolyse, éventuellement une distillation ou une concentration sous vide pourra s'avérer nécessaire.

Le procédé de réduction d'un composé organique insaturé choisi dans le groupe formé par les aldéhydes, les cétones, les imines, les acides carboxyliques, les amides, et les esters, avec un formiate de bore de formule (I) selon l'invention peut également être utilisé pour la fabrication de vitamines, de produits pharmaceutiques, de colles, de fibres acryliques, de cuirs synthétiques, de pesticides.

Ainsi, l'invention concerne également un procédé de fabrication de vitamines, de produits pharmaceutiques, de colles, de fibres acryliques, de cuirs synthétiques, de pesticides comprenant une étape de réduction d'un composé organique insaturé choisi dans le groupe formé par les aldéhydes, les cétones, les imines, les acides carboxyliques, les amides, et les esters, avec un formiate de bore de formule (I) selon le procédé de l'invention.

Les différentes utilisations décrites pour le procédé de réduction des composés organiques insaturés précités

par les formiates de formule (I) selon l'invention, s'appliquent de la même manière aux formiates de bore de formule (Ia).

D'autres avantages et caractéristiques de la présente invention apparaîtront à la lecture des exemples ci-dessous donnés à titre illustratif et non limitatif et les figures annexées :

- la Figure 1 représente détaille le procédé industriel de préparation du tétraborohydrure de sodium $NaBH_4$ (TBHS) à partir du borax, source naturelle de l'élément bore ;
- la Figure 2 représente la dismutation de l'acide formique en $CO_2$ et méthanol ;
- la Figure 3 représente deux réactions de décomposition de l'acide formique qui sont en compétition avec la réaction de dismutation de l'acide formique en méthanol et $CO_2$. Ces deux réaction sont : la déshydrogénation de l'acide formique en $CO_2$ et $H_2$ (Équation (4)) et la déshydratation de l'acide formique en CO et $H_2O$ (Équation (5)) ;
- la Figure 4 représente des exemples de carbènes N-hétérocycliques ;
- la Figure 5 représente la préparation de l'acide formique par une électro-réduction à $2e^-$ ou une hydrogénation catalytique du $CO_2$ ;
- la Figure 6 représente la réduction des aldéhydes, des cétones, des acides carboxyliques et des esters, en alcools ; les imines en aminés ; et les amides en amines ou en alcools avec $R^1$, $R^2$, et $R^3$ comme définis précédemment ;
- la Figure 7 représente un exemple de réduction d'un aldéhyde en alcool primaire selon le procédé de l'invention, ainsi que le cas particulier de dismutation de formiate de bore de formule (I) ou (Ib) en méthanol, en formaldéhyde et une amine méthylée ;
- la Figure 8 montre le clivage hétérolytique de $H_2$ avec une FLP, à savoir $\{B(C_6F_5)_3$ + *t*-$Bu_3P\}$, pour former un sel, à savoir un phosphonium ($PH^+$) de boratohydrure ($BH^-$), suivi de l'hydrogénation du $CO_2$ par ledit sel pour conduire au formiate de bore correspondant.

**EXEMPLES**

Un ensemble de résultats est présenté ci-dessous, donnant des exemples de synthèses de formiates de bore et de leurs applications dans des réactions de réduction d'aldéhydes mais aussi de dismutation en méthoxyboranes et de méthylation des amines. Les rendements sont obtenus par intégration des signaux du produit réduit par rapport à ceux du mésitylène ou du diphénylméthane ($Ph_2CH_2$) utilisés comme étalon interne.

Dans le cas de la préparation du méthanol par la réaction de dismutation de l'acide formique, les rendements sont calculés en respectant la stœchiométrie de la réaction de dismutation à savoir qu'au mieux, 3 moles d'acide formique donnent au maximum 1 mol de méthanol.

Rendement en produit de réduction :

$$\rho(réduction) = \frac{n(produit\ réduit)}{n_0(substrat\ à\ réduire)}$$

Rendement en méthanol :

$$\rho(MeOH) = \frac{3 \times n(MeOH)}{n_0(HCOOZ)}$$

Rendement en méthoxyborane :

$$\rho(MeOBR^1R^2) = \frac{3 \times n(MeOBR^1R^2)}{n_0(HCOOZ)}$$

Rendement en amine méthylée :

$$\rho(MeNR^1R^2) = \frac{n(MeNR^1R^2)}{n_0(HNR^1R^2)}$$

- *n*(*produit réduit*) : quantité de matière en produit de réaction déterminé par RMN $^1H$ par rapport au mésitylène
- $n_0$(*substrat*) : quantité de matière en substrat réductible introduit initialement.
- *n*(*MeOH*) : quantité de matière déterminé par RMN $^1H$ par rapport au mésitylène.

- $n_0(MeOBR^1R^2)$ : quantité de matière en méthoxyborane déterminé par RMN $^1H$ par rapport au mésitylène.
- $n(MeNR^1R^2)$ : quantité de matière en amine méthylée déterminé par RMN $^1H$ par rapport au mésitylène ou au diphénylméthane.
- $n_0(HNR^1R^2)$ : quantité de matière en amine introduit initialement sous forme d'amine libre ou en tant que Z dans les formiates de bore de formule (Ib)
- $n_0(HCOOZ) = n_0(HCOOH) + n_0(HCOOM)$: quantité de matière totale en acide formique (HCOOH) et ses dérivés (HCOOM, M tel que défini plus haut) introduite initialement.

**Protocole de préparation des formiates de bore de formule (I)**

Le formiate de bore de formule (I) peut être préparé selon le protocole expérimental suivant :

1. Sous atmosphère inerte, en boîte à gants, l'organoborane de formule (II), l'acide formique ou l'un de ses dérivés, ou un mélange d'acide formique et d'au moins un de ses dérivés et éventuellement, le solvant et/ou la base et/ou l'additif sont introduits dans un tube de Schlenk qui est ensuite scellé par un robinet J. Young. L'ordre d'introduction des réactifs n'a pas d'importance.
2. Le mélange réactionnel est ensuite agité à une température comprise entre 0 et 30°C (préférentiellement à la température ambiante, c'est-à-dire 20 ± 5°C) jusqu'à la conversion totale de l'acide formique (de 5 minutes à 24 heures de réaction). Le suivi de la réaction est assuré par RMN du proton $^1H$ et/ou $^{13}C$ et/ou $^{11}B$.
3. Lorsque la réaction est terminée (ce qui correspond à l'apparition du signal caractéristique des formiates de bore en RMN $^{11}B$), le solvant ainsi que les composés volatils sont évaporés sous vide ($10^{-2}$ mbar). Dans certains cas, il est également possible de précipiter le formiate de bore par ajout du pentane au milieu réactionnel, et celui-ci est alors récupérer par filtration sur Büchner.
4. Le résidu obtenu après évaporation des volatils est ensuite lavé avec du pentane et de l'éther pour obtenir un solide qui est ensuite séché sous vide dynamique pendant au moins 2 h.

**EXEMPLE 1 : Synthèse de $[Et_3NH^+, Cy_2B(OCHO)_2^-]$ ($R_1 = R_2 = Cy$ ; X = -OCHO, $Z = Et_3NH^+$)**

Le dicyclohexylborane $Cy_2BH$ est synthétisé à partir d'une procédure décrite dans la littérature (A. Abiko, Org. Synth. 2002, 79, 103) et est utilisé sans purification particulière.

Dans un ballon de 25 mL, muni d'un barreau aimanté et d'un robinet J-Young, est ajouté $Cy_2BH$ (481 mg, 2.7 mmol, 1 équiv.) et 5 mL de toluène. La suspension obtenue est agitée jusqu'à complète dissolution du solide puis l'acide formique (204 μL, 5.4 mmol, 2 équiv) est ajouté à l'aide d'une seringue suivi par la $NEt_3$ (377 μL, 2.7 mmol, 1 equiv) en une seule fois. Un fort dégagement d'hydrogène gaz est observé. La réaction est alors agitée pendant 2 h à température ambiante puis le solvant est évaporé à sec pour laisser une huile très visqueuse. Après de multiples ajouts de pentane et trituration de l'huile dans de l'hexane, l'huile cristallise et un solide blanc est obtenu, celui-ci est alors récupéré par filtration et lavé au pentane (3 x 2 mL) et à l'éther (3 x 2mL). Le solide blanc ainsi récupéré est séché sous pression réduite pour obtenir **$[Et_3NH^+, Cy_2B(OCHO)_2^-]$** (901 mg) avec un rendement de 90 %.
**$^1H$ NMR (200 MHz, $CD_3CN$)** δ 8.77 (s, 1H, N<u>H</u>), 8.29 (s, 2H, <u>H</u>C(O)O), 3.13 (t, *J*= 7.2 Hz, 6H), 1.65 (d, *J* = 4.3 Hz, 4H), 1.50 (d, *J* = 12.8 Hz, 4H), 1.24 (t, *J* = 7.3 Hz, 9H), 1.12 (d, *J* = 7.6 Hz, 4H), 1.01 - 0.73 (m, 4H), 0.48 (tt, *J* = 12.0 Hz, 2H, C<u>H</u>-B) ppm.
**$^{13}C$ NMR (50 MHz, $CD_3CN$)** δ 166.43, 47.39, 29.38, 28.50, 9.06. ppm.
**$^{11}B$ NMR** (64 MHz, $CD_3CN$) δ 11.17 ppm.
**Analyse élémentaire:** calc. (%) pour $C_{20}H_{40}BNO_4$ (369.30 g.mol$^{-1}$): C 65.04, H 10.92, N 3.79; trouvé : C 63.05, H 11.03, N 3.41.

**EXEMPLE 2 : Synthèse de $[Et_3NH^+, BBN(OCHO)_2^-]$ ($R_1 = R_2 = BBN$ ; X = -OCHO, $Z = Et_3NH^+$)**

Dans un ballon de 100 mL, muni d'un barreau aimanté et d'un robinet J-Young, est ajouté du dimère 9-BBN (1.95 g, 7.98 mmol, 0.5 équiv.) et 20 mL de toluène. La suspension obtenue est agitée jusqu'à complète dissolution du solide puis l'acide formique (1,2 mL, 31,92 mmol, 2 équiv) est ajouté à l'aide d'une seringue suivi par la triéthylamine (2,2 mL, 15,96 mmol, 1 équiv) en une seule fois. Un fort dégagement d'hydrogène gaz est observé. La réaction est alors agitée pendant 2 h à température ambiante puis du pentane (5 mL) est ajouté puis le solvant est évaporé à sec pour laisser une huile très visqueuse. L'huile est triturée dans de l'hexane jusqu'à obtention d'un solide, et le solide blanc obtenu est alors récupéré par filtration et lavé au pentane (3 x 4 mL) et à l'éther (3 x 4mL). Le solide blanc ainsi récupéré est séché sous pression réduite pour obtenir **$[Et_3NH^+, BBN(OCHO)_2^-]$** avec un rendement de 92 %.
**$^1H$ NMR (200 MHz, $CD_3CN$)** δ 8.60 (bs, 1H), 8.44 (s, 2H), 3.14 (q, *J* = 7.3 Hz, 6H), 1.88 - 1.36 (m, 12H), 1.24 (t, *J* = 7.3 Hz, 9H), 0.75 (bs, 2H) ppm.

**$^{13}C$ NMR (50 MHz, $CD_3CN$)** δ 167.84, 47.46, 32.09, 25.58, 9.04 ppm.
**$^{11}B$ NMR** (64 MHz, $CD_3CN$) δ 8.98 ppm.
**Analyse élémentaire** : calc (%) for $C_{16}H_{32}BNO_4$ (313.25 g.mol$^{-1}$): C 61.35, H 10.30, N 4.47; found: C 58.29, H 10.13, N 4.28.

**EXEMPLE 3 : Synthèse de [*i*-$Pr_2EtNH^+$, $BBN(OCHO)_2^-$] ($R_1$ = $R_2$ = BBN ; X = - OCHO, Z = *i*-$Pr_2EtNH^+$)**

Par une procédure similaire à celle décrite pour [$Et_3NH^+$, $BBN(OCHO)_2^-$] en remplaçant la triéthylamine par la diisopropyléthylamine (DIPEA), le solide blanc **[*i-Pr*$_2$EtNH$^+$, $BBN(OCHO)_2^-$]** est obtenu avec un rendement de 76 % (766 mg).
**$^1H$ NMR (200 MHz, $CD_3CN$)** δ 8.44 (s, 2H), 7.87 (bs, 1H), 3.68 (h, 2H), 3.15 (q, *J* = 7.2 Hz, 2H), 1.88 - 1.15 (m, 27H), 0.74 (s, 2H).
**$^{13}C$ NMR (50 MHz, $CD_3CN$)** δ 167.75, 55.47, 43.61, 32.08, 25.60, 18.57, 17.26, 12.85.
**$^{11}B$ NMR** (64 MHz, $CD_3CN$) δ 9.03.
**Analyse élémentaire** : calc (%) for $C_{18}H_{36}BNO_4$ (341.29 g.mol$^{-1}$): C 63.35, H 10.63, N 4.10; found: C 62.80, H 10.67, N 3.99.

**EXEMPLE 4 : Synthèse de [$Cy_3PH^+$, $BBN(OCHO)_2^-$] ($R_1$ = $R_2$ = BBN ; X = -OCHO, Z = $Cy_3PH^+$)**

Par une procédure similaire à celle décrite pour [$Et_3NH^+$, $BBN(OCHO)_2^-$] en remplaçant la triéthylamine par la tricyclohexylphosphine ($Cy_3P$, 20% massique dans le toluène), le solide blanc **[$Cy_3PH^+$, $BBN(OCHO)_2^-$]** est obtenu avec un rendement de 73 % (729 mg).
**$^1H$ NMR (200 MHz, $CD_3CN$)** δ 8.44 (s, 2H), 2.51 (q, *J* = 11.7 Hz, 3H), 1.87 - 1.22 (m, 43H), 0.72 (s, 2H).
**$^{13}C$ NMR (50 MHz, $CD_3CN$)** δ 166.85, 32.22, 28.88, 28.46, 28.12, 26.92, 26.66, 25.79.
**$^{11}B$ NMR** (64 MHz, $CD_3CN$) δ 8.46
**$^{31}P$ NMR** (81 MHz, $CD_3CN$) δ 32.21 (s).

**EXEMPLE 5 : Synthèse de [$TMPH^+$, $BBN(OCHO)_2^-$] ($R_1$ = $R_2$ = BBN ; X = -OCHO, Z = $TMPH^+$)**

Par une procédure similaire à celle décrite pour [$Et_3NH^+$, $BBN(OCHO)_2^-$] en remplaçant la triéthylamine par la 2,2,6,6-tétraméthylpipéridine (TMP), le solide blanc **[$Cy_3PH^+$, $BBN(OCHO)_2^-$]** est obtenu avec un rendement de 89 % (890 mg). Le solide blanc obtenu est purifié par recristallisation dans l'acétonitrile.
**$^1H$ NMR (200 MHz, $CD_3CN$)** δ 8.44 (s, 2H), 7.87 (bs, 1H), 3.68 (h, 2H), 3.15 (q, *J* = 7.2 Hz, 2H), 1.88 - 1.15 (m, 27H), 0.74 (s, 2H).
**$^{13}C$ NMR (50 MHz, $CD_3CN$)** δ 169.13, 60.45, 37.22, 33.97, 29.24, 27.52, 18.51.
**$^{11}B$ NMR** (64 MHz, $CD_3CN$) δ 8.30.
**Analyse élémentaire** : calc (%) for $C_{19}H_{36}BNO_4$ (353.31 g.mol$^{-1}$): C 64,59, H 10.27, N 3,96; found: C 64,15, H 10.31, N 4,02.

**Protocole de base pour la réduction de composés organiques insaturés avec les formiates de bore de formule (I)**

Pour la réduction des composés organiques présentant une fonction insaturée réductible, les formiates de bore sont mis à réagir avec le substrat à réduire (si celui-ci n'est pas l'acide formique lui-même) ou à fonctionnaliser. Celui-ci peut être un composé organique présentant au moins une fonction insaturée réductible : aldéhydes, cétones, imines, acide carboxyliques, amides, esters. Il est également envisageable de rajouter une amine (primaire ou secondaire) pour effectuer la méthylation de celle-ci lorsque le substrat est l'acide formique lui-même (conditions de dismutation).
Ces réductions peuvent être effectuées selon le protocole suivant :

5. Sous atmosphère inerte, en boîte à gants, le formiate de bore de formule générale (I) (0,01 à 2 équivalent molaire par rapport au substrat), le substrat à réduire, et éventuellement l'acide formique et/ou l'un de ses dérivés, le solvant et/ou la base et/ou l'additif sont introduits dans un tube de Schlenk qui est ensuite scellé par un robinet J. Young. L'ordre d'introduction des réactifs n'a pas d'importance.
6. Le Schlenk est ensuite chauffé à une température comprise entre 25 et 150 °C (préférentiellement > 80°C) jusqu'à la conversion totale de l'acide formique (de 5 minutes à 48 heures de réaction). Le suivi de la réaction est assuré par RMN du proton $^1H$ et/ou $^{13}C$ et/ou $^{11}B$ et/ou $^{31}P$.
7. Lorsque la réaction est terminée (ce qui correspond à la disparition des signaux caractéristiques des protons du formiate H-COO$^-$ en RMN $^1H$), la pression dans le tube est libérée. A ce stade, le traitement des réactions dépend de la nature du produit réduit obtenu. Les exemples ci-dessous permettent d'apprécier les différences de traitement

des réactions de réduction en fonction des produits obtenus.

**EXEMPLE 6 : Formation du méthanol**

Selon le protocole général présenté ci-dessus, un tube RMN muni d'une valve J-Young est rempli avec **[$Et_3NH^+$, $BBN(OCHO)_2^-$]** (0.125 mmol). Ce dernier est alors dissous dans de l'acétonitrile (0.30 mL), le tube est scellé et chauffé à 130°C pendant 19 h. Les composés volatils (MeCN et $NEt_3$) sont ensuite évaporés sous vide ($10^{-1}$ à $10^{-2}$ mbar), le résidu solide visqueux obtenu est alors dissous dans du THF et $H_2O$ (5-10 equiv. par rapport au formiate de bore intialement introduit) est ajouté au mélange réactionnel. La solution est agitée 30 minutes à 1 heure à température ambiante (20 ± 5 °C). Le méthanol volatil est ensuite récupéré dans un autre tube de Schlenk par transfert sous pression réduite. On obtient ainsi une solution aqueuse de méthanol avec un rendement de 49 %.

Le tableau ci-dessous rassemble quelques résultats permettant l'obtention de méthanol avec différents formiates de bore et après hydrolyse aqueuse (effectuée telle que décrite ci-dessus).

| Réactif (mmol) | Solvant | Température (°C) | Temps (h) | Rendement (%) |
|---|---|---|---|---|
| [$Et_3NH^+$,$BBN(OCHO)_2^-$] (0,125) | MeCN | 130 | 19 | 49 |
| [$Et_3NH^+$, $Cy_2B(OCHO)_2^-$] (0,125) | MeCN | 120 | 7 | 31 |
| [*i*-$Pr_2EtNH^+$,$BBN(OCHO)_2^-$] | MeCN | 130 | 7 | 50 |
| [$Na^+$, $BBN(OCHO)_2^-$] + 15-C-5 | MeCN | 130 | 24 | 58 |

**EXEMPLE 7 : Formation d'amine méthylées**

Selon le protocole général présenté ci-dessus, un tube RMN muni d'une valve J-Young est rempli avec **[$TMPH^+$, $BBN(OCHO)_2^-$]** (0.125 mmol). Ce dernier est alors dissous dans de l'acétonitrile (0.30 mL), le tube est scellé et chauffé à 130°C pendant 23 h. Les composés volatils sont ensuite transférés sous vide ($10^{-1}$ à $10^{-2}$ mbar) dans un autre tube de Schlenk. On obtient alors une solution composée de TMP et de TMP-$CH_3$ (TMP méthylée, 23 % de rendement) dans l'acétonitrile. Ces dernières peuvent être séparées par distillation sous pression réduite ($T_{eb}$(760 mmHg ou 1,0132472 bar) = 152°C et 187°C respectivement). Il est à noter que la TMP régénérée en fin de réaction peut être réutilisée pour former **[$TMPH^+$, $BBN(OCHO)_2^-$]** par le procédé d'invention.

**EXEMPLE 8 : Formation de formaldehyde**

Selon le protocole général présenté ci-dessus, un tube RMN muni d'une valve J-Young est rempli avec **[$Cy_3PH^+$, $BBN(OCHO)_2^-$]** (0.125 mmol). Ce dernier est alors dissous dans de l'acétonitrile (0.30 mL), le tube est scellé et chauffé à 130°C pendant 5,5 h. A ce stade, le formaldehyde est obtenu sélectivement (le méthanol n'est pas observé) sous forme de l'acétal $Cy_3PCH_2OBBN(OCHO)$. Les composés volatils sont ensuite transférés sous vide ($10^{-1}$ à $10^{-2}$ mbar) dans un autre tube de Schlenk et le résidu solide obtenu est alors dissous dans du THF et $H_2O$ (5-10 equiv. par rapport au formiate de bore intialement introduit) est ajouté au mélange réactionnel. La solution est agitée 30 minutes à 1 heure à température ambiante (20 ± 5 °C). On obtient alors une solution aqueuse de formaldéhyde (28 % de rendement) qui doit être utilisée telle quelle au besoin.

**EXEMPLE 9 : Réduction du benzaldehyde en alcool benzylique**

Selon le protocole général présenté ci-dessus, un tube RMN muni d'une valve J-Young est rempli avec **[$Et_3NH^+$, $BBN(OCHO)_2^-$]** (0.10 mmol), du benzaldéhyde (0.05 mmol) et de l'acétonitrile (0.30 mL), le tube est scellé et chauffé à 130°C pendant 5 h. Les composés volatils (MeCN et $NEt_3$) sont ensuite évaporés sous vide ($10^{-1}$ à $10^{-2}$ mbar), le résidu solide visqueux obtenu est alors dissous dans du THF et $H_2O$ (5-10 equiv. par rapport au formiate de bore intialement introduit) est ajouté au mélange réactionnel. La solution est agitée 30 minutes à 1 heure à température ambiante (20 ± 5 °C). L'alcool benzylique formé est ensuite récupéré par transfert sous pression réduite dans un autre tube de Schlenk. On obtient ainsi une solution d'alcool benzylique avec un rendement de 99 %. Ce dernier peut être finalement récupéré et purifié par distillation ($T_{eb}$(760 mmHg ou 1,0132472 bar) = 203°C).

**EXEMPLE 10 : Réduction du cinnamaldéhyde en alcool cinnamique**

Selon le protocole général présenté ci-dessus, un tube RMN muni d'une valve J-Young est rempli avec **[$Et_3NH^+$,**

**$BBN(OCHO)_2^-$]** (0,10 mmol), du cinnamaldéhyde (0,05 mmol) et de l'acétonitrile (0,30 mL), le tube est scellé et chauffé à 130°C pendant 5 heures. Les composés volatils (MeCN et $NEt_3$) sont ensuite évaporés sous vide ($10^{-1}$ à $10^{-2}$ mbar), le résidu solide visqueux obtenu est alors dissous dans du THF et $H_2O$ (5-10 équivalents par rapport au formiate de bore initialement introduit) est ajouté au mélange réactionnel. La solution est agitée 30 minutes à 1 heure à température ambiante (20 $\pm$ 5°C). L'alcool cinnamique formé (rendement de 80 %) peut être finalement récupéré et purifié par distillation ($T_{eb}$(760 mmHg ou 1,0132472 bar) = 250°C).

**EXEMPLE 11** : **Réduction du 4-chlorobenzaldéhyde en alcool 4-chlorobenzylique**

Selon le protocole général présenté ci-dessus, un tube RMN muni d'une valve J-Young est rempli avec **[$Et_3NH^+$, BBN(OCHO)2$^-$]** (0,10 mmol), du 4-chlorobenzaldéhyde (0,05 mmol) et de l'acétonitrile (0,30 mL), le tube est scellé et chauffé à 130°C pendant 5 heures. Les composés volatils (MeCN et $NEt_3$) sont ensuite évaporés sous vide ($10^{-1}$ à $10^{-2}$ mbar), le résidu solide visqueux obtenu est alors dissous dans du THF et $H_2O$ (5-10 équivalents par rapport au formiate de bore initialement introduit) est ajouté au mélange réactionnel. La solution est agitée 30 minutes à 1 heure à température ambiante (20 $\pm$ 5°C). L'alcool alcool 4-chlorobenzylique formé (rendement de 99 %) peut être finalement récupéré et purifié par distillation ($T_{eb}$(760 mmHg ou 1,0132472 bar) = 234°C).

**Remarque** : Il est à noter que les réactions conduisant aux amines méthylées ou au formaldéhyde ne forment que ceux-ci comme produits de réduction. Autrement dit, les rendements moyens observés pour ces réactions peuvent être corrigés en tenant compte du fait que les autres produits sont les produits de départ (amine ou formiate) ou les gaz $CO_2$ et $H_2$. Dans tous les cas, ceux-ci peuvent être réutilisés dans le procédé pour préparer l'acide formique ou le formiate de bore de formule (I).

## Revendications

1. Procédé de réduction de composés organiques insaturés choisis dans le groupe formé par les aldéhydes, les cétones, les imines, les acides carboxyliques, les amides, et les esters, **caractérisé en ce que** l'on fait réagir ledit composé organique insaturé avec un formiate de bore de formule (I)

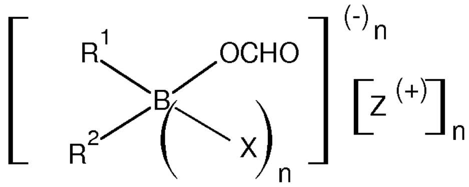

(I)

dans laquelle

- $R^1$ et $R^2$, indépendamment l'un de l'autre, sont choisis un groupe hydroxyle, un groupe alcoxy, un groupe alkyle, un groupe alcényle, un groupe alcynyle, un groupe aryle, un groupe hétéroaryle, un groupe hétérocyclique, un atome d'halogène, un groupe silyle, un groupe siloxy, un groupe phosphino, et un groupe amino, lesdits groupes alkyle, alcényle, alcynyle, alcoxy, silyle, siloxy, aryle, phosphino et amino étant éventuellement substitués ; ou
- $R^1$ et $R^2$, pris ensemble avec l'atome de bore auquel ils sont liés, forment un hétérocycle éventuellement substitué ;
- X est choisi dans le groupe formé par un atome d'halogène, -OCHO et un groupe sulfonate de formule-$OSO_2R^7$, dans lequel $R^7$ est choisi parmi un groupe méthyle ($CH_3$), un groupe trifluorométhyle ($CF_3$), un groupe toluène ($p$-$CH_3C_6H_4$) ou un groupe benzène ($C_6H_5$),
- Z est un cation choisi dans le groupe formé par une base organique protonnée ayant un pKa supérieur à 3,7 choisie dans le groupe formé par le triéthylammonium ($HNEt_3^+$), le di-isopropyléthylammonium ($i$-$Pr_2EtNH^+$), le 2,2,6,6-tétramethylpipéridinium ($TMPH^+$), et le tricyclohexylphosphonium ($HPCy_3^+$) ; $Na^+$ ; $Li^+$ ; $K^+$ ; $Cs^+$ ; le tétraphénylphosphonium ($PPh_4^+$) ; le tétraméthylammonium ($NMe_4^+$) ; le tétraéthylammonium ($NEt_4^+$) ; le tétrabutylammonium ($NBu_4^+$) et le tétraphénylammonium ($NPh_4^+$) ;
- n est un nombre entier égal à 1 ;

en présence d'un solvant et éventuellement d'une base organique ou inorganique,

étant entendu que les formiates de bore de formule (I) dans lesquelles n est égal à 1, ne sont pas des paires de Lewis frustrées.

**2.** Procédé selon la revendication 1, **caractérisé en ce que**

- les aldéhydes, les cétones, les acides carboxyliques et les esters, sont réduits en alcools ;
- les imines sont réduites en aminés ; et
- les amides sont réduits en amines ou en alcools.

**3.** Procédé selon l'une des revendications 1 ou 2, **caractérisé en ce que** dans le formiate de bore de formule (I),

- $R^1$ et $R^2$, indépendamment l'un de l'autre, sont choisis dans le groupe formé par un groupe alkyle comprenant 1 à 12 atomes de carbone ; un aryle comprenant 6 à 20 atomes de carbone, lesdits groupes alkyle et aryles étant éventuellement substitués.

**4.** Procédé selon l'une des revendications 1 ou 2, **caractérisé en ce que** dans le formiate de bore de formule (I),

- $R^1$ et $R^2$ pris ensemble avec l'atome de bore auquel ils sont liés, forment un hétérocycle comportant de 5 à 10 membres, ledit hétérocycle étant éventuellement substitué.

**5.** Procédé selon l'une quelconque des revendications 1 à 4, **caractérisé en ce que** Z est un cation choisi dans le groupe formé par le triéthylammonium ($HNEt_3^+$), le di-isopropyléthylammonium ($i$-$Pr_2EtNH^+$), le 2,2,6,6-tétraméthylpipéridinium ($TMPH^+$), le tricyclohexylphosphonium ($HPCy_3^+$), et $Na^+$.

**6.** Procédé selon l'une quelconque des revendications 1 à 5, **caractérisé en ce que** les formiates de bore de formule (I) sont [$Et_3NH^+$, $BCy_2(OCHO)_2^-$], [$i$-$Pr_2EtNH^+$, $BCy_2(OCHO)2^-$], [$Et_3NH^+$, $n$-$Bu_2B(OCHO)_2^-$], [$Et_3NH^+$, $BBN(OCHO)_2^-$], [$i$-$Pr_2EtNH^+$, $BBN(OCHO)_2^-$], [$Na^+$, $BBN(OCHO)_2^-$], [$Cy_3PH^+$, $BBN(OCHO)_2^-$] et [$TMPH^+$, $BBN(OCHO)_2^-$].

**7.** Procédé selon l'une quelconque des revendications 1 à 6, **caractérisé en ce qu'**il a lieu dans un ou un mélange d'au moins deux solvants choisis dans le groupe formé par :

- les éthers choisis parmi l'éther diéthylique, le THF, le diglyme, le 1,4-dioxane ;
- les hydrocarbures choisis parmi le benzène, ou le toluène ;
- les solvants azotés choisis parmi la pyridine, ou l'acétonitrile ;
- les sulfoxydes choisis parmi le diméthylesulfoxyde ;
- les halogénures d'alkyle choisis parmi le chloroforme, ou le chlorure de méthylène ; et
- un fluide supercritique choisi parmi le $CO_2$ supercritique.

**8.** Procédé selon l'une quelconque des revendications 1 à 7, **caractérisé en ce que** la quantité des composés organiques insaturés à réduire est de 0,5 à 2 équivalents molaires, par rapport au formiate de bore de formule (I).

**9.** Procédé selon l'une quelconque des revendications 1 à 8, **caractérisé en ce qu'**il a lieu en présence d'un additif choisi parmi :

- les éthers couronnes choisis dans le groupe formé par le 12-couronne-4, le 15-couronne-5, le 18-couronne-6, le dibenzo-18-couronne-6, le benzo-18-couronne-6, le benzo-15-couronne-5, et le dibenzo-15-couronne-5 ;
- les aza-couronnes choisis dans le groupe formé par le 1,4,7,10-tétraazacyclododécane (cyclène), le 1,4,7,10,13,16-hexaazacyclooctadécane (hexacyclène), et le diaza-18-couronne-6 ;
- les thioéthers couronnes choisis dans le groupe formé par le 1,5,9,13-tétrathiacyclohexadécane (16-Ane-$S_4$), et le 1,4,7,10,13,16-hexathiacyclooctadécane (18-Ane-$S_6$).

**10.** Procédé selon la revendication 9, **caractérisé en ce que** la quantité de l'additif est de 1 à 2 équivalents molaires, bornes incluses, par rapport au formiate de bore de formule (I).

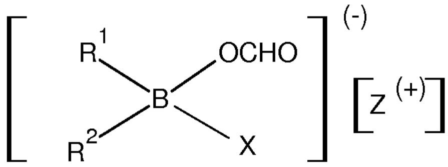

**11.** Utilisation d'un procédé de réduction de composés organiques insaturés choisis dans le groupe formé par les aldéhydes, les cétones, les imines, les acides carboxyliques, les amides, et les esters avec les formiates du bore de formule (I) selon l'une quelconque des revendications 1 à 10,

- pour la préparation du méthanol, de amine méthylées de formule $R^1R^2N\text{-}CH_3$, de formaldéhyde et d'alcools de formule $R^1CH_2OH$ avec $R^1$ et $R^2$ tels que définis dans l'une des revendications 1, 3 et 4 ;
- pour la préparation de réactifs pour les réactions de couplage Suzuki,
- dans la fabrication de vitamines, de produits pharmaceutiques, de colles, de fibres acryliques, de cuirs synthétiques, de pesticides.

**12.** Procédé de préparation de méthanol comprenant

(i) une étape de réduction de l'acide formique ou de l'un de ses esters de formule $HCO_2R^8$ dans laquelle $R^8$ est choisi parmi un atome d'hydrogène, un groupe alkyle, ou un groupe aryle, avec un formiate de bore de formule (I) selon le procédé de l'une quelconque des revendications 1 à 10, et éventuellement
(ii) une étape d'hydrolyse.

**13.** Procédé de préparation d'amines méthylées de formule $R^1R^2N\text{-}CH_3$ avec $R^1$ et $R^2$ tels que définis dans les revendications 1, 3 ou 4, comprenant

(i) une étape de réduction de l'acide formique en présence d'une amine primaire ou d'une amine secondaire, avec un formiate de bore de formule (I) selon le procédé de l'une quelconque des revendications 1 à 10, et
(ii) une distillation ou une concentration sous vide ou une chromatographie sur colonne.

**14.** Procédé de préparation de formaldéhyde, comprenant une étape de réduction de l'acide formique ou de l'un de ses esters de formule $HCO_2R^8$ dans laquelle $R^8$ est choisi parmi un atome d'hydrogène, un groupe alkyle, ou un groupe aryle, avec un formiate de bore de formule (I) selon le procédé de l'une quelconque des revendications 1 à 10, et éventuellement
(ii) une étape d'hydrolyse.

**15.** Procédé de préparation d'un alcool de formule $R^1CH_2OH$ avec $R^1$ tel que défini tels que définis dans les revendications 1, 3 ou 4, comprenant

(i) une étape de réduction d'un composé organique insaturé choisi dans le groupe formé par les aldéhydes, les acides carboxyliques et les esters avec un formiate de bore de formule (I) selon le procédé de l'une quelconque des revendications 1 à 10, et éventuellement
(ii) une étape d'hydrolyse.

**Patentansprüche**

**1.** Verfahren zur Reduktion ungesättigter organischer Verbindungen, die aus der Gruppe ausgewählt werden, die aus den Aldehyden, den Ketonen, den Iminen, den Carbonsäuren, den Amiden und den Estern gebildet wird, **dadurch gekennzeichnet, dass** die ungesättigte organische Verbindung dazu gebracht wird, mit einem Borformiat der Formel (I) zu reagieren

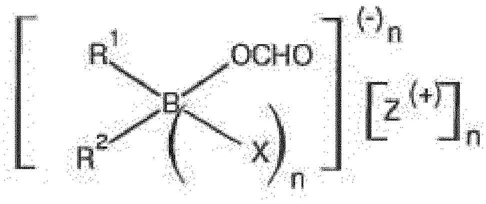

(I)

wobei

- $R^1$ und $R^2$ unabhängig voneinander aus einer Hydroxylgruppe, eine Alkoxygruppe, einer Alkylgruppe, einer Alkenylgruppe, einer Alkynylgruppe, einer Arylgruppe, einer Heteroarylgruppe, einer heterocyclischen Gruppe, einem Halogenatom, einer Silylgruppe, einer Siloxygruppe, einer Phosphinogruppe und einer Aminogruppe ausgewählt werden, wobei die Alkyl-, Alkenyl-, Alkynyl-, Alkoxy-, Silyl-, Siloxy-, Aryl-, Phosphino- und Aminogruppen gegebenenfalls substituiert sind; oder
- $R^1$ und $R^2$ mit dem Boratom, an das sie gebunden sind, zusammengenommen einen gegebenenfalls substituierten Heterocyclus bilden;
- X aus der Gruppe ausgewählt wird, die aus einem Halogenatom, --OCHO und einer Sulfonatgruppe der Formel $-OSO_2R^7$ gebildet wird, wobei $R^7$ aus einer Methylgruppe ($CH_3$), einer Trifluormethylgruppe ($CF_3$), einer Toluolgruppe ($p$-$CH_3C_6H_4$) oder einer Benzolgruppe ($C_6H_5$) ausgewählt wird,
- Z ein Kation ist, das aus der Gruppe ausgewählt wird, die aus einer protonierten organischen Base gebildet wird, die ein pKa größer als 3,7 aufweist, die aus der Gruppe ausgewählt wird, die aus dem Triethylammonium ($HNEt_3^+$), dem Di-isopropylethylammonium ($i$-$Pr_2EtNH^+$), dem 2,2,6,6-Tetramethylpiperidinium ($TMPH^+$) und dem Tricyclohexylphosphonium ($HPCy_3^+$); $Na^+$; $Li^+$; $K^+$; $Cs^+$; dem Tetraphenylphosphonium ($PPh_4^+$), dem Tetramethylammonium ($NMe_4^+$) dem Tetraethylammonium ($NEt_4^+$); dem Tetrabutylammonium ($NBu_{4+}$) und dem Tetraphenylammonium ($NPh_4^+$) gebildet wird;
- n eine ganze Zahl gleich 1 ist;

bei Vorhandensein eines Lösungsmittels und gegebenenfalls einer organischen oder anorganischen Base, wobei es sich versteht, dass die Borformiate der Formel (I), bei denen n gleich 1 ist, keine frustrierten Lewis-Paare sind.

2. Verfahren nach Anspruch 1, **dadurch gekennzeichnet, dass**

- die Aldehyde, die Ketone, die Carbonsäuren und die Ester zu Alkoholen reduziert werden;
- die Imine zu Aminen reduziert werden; und
- die Amide zu Aminen oder zu Alkoholen reduziert werden.

3. Verfahren nach einem der Ansprüche 1 oder 2, **dadurch gekennzeichnet, dass** im Borformiat der Formel (I)

- $R^1$ und $R^2$, unabhängig voneinander, aus der Gruppe ausgewählt werden, die aus einer Alkylgruppe, 1 bis 12 Kohlenstoffatome umfassend; einem Aryl, 6 bis 20 Kohlenstoffatome umfassend, gebildet wird, wobei die Alkyl- und Arylgruppen gegebenenfalls substituiert sind.

4. Verfahren nach einem der Ansprüche 1 oder 2, **dadurch gekennzeichnet, dass** im Borformiat der Formel (I)

- $R^1$ und $R^2$ mit dem Boratom, an das sie gebunden sind, zusammengenommen einen Heterocyclus bilden, der 5 bis 10 Teile einschließt, wobei der Heterocyclus gegebenenfalls substituiert ist.

5. Verfahren nach einem der Ansprüche 1 bis 4, **dadurch gekennzeichnet, dass** Z ein Kation ist, das aus der Gruppe ausgewählt wird, die aus dem Triethylammonium ($HNEt_3^+$), dem Di-isopropylethylammonium ($i$-$Pr_2EtNH^+$), dem 2,2,6,6-Tetramethylpiperidinium ($TMPH^+$), dem Tricyclohexylphosphonium ($HPCy_3^+$) und $Na^+$ gebildet wird.

6. Verfahren nach einem der Ansprüche 1 bis 5, **dadurch gekennzeichnet, dass** die Borformiate der Formel (I) [$Et_3NH^+$, $BCy_2(OCHO)_2^-$], [$i$-$Pr_2EtNH^+$, $BCy_2(OCHO)_2^-$], [$Et_3NH^+$, $n$-$Bu_2B(OCHO)_2^-$], [$Et_3NH^+$, $BBN(OCHO)_2^-$ ], [$i$-$Pr_2EtNH^+$, $BBN(OCHO)_2^-$], [$Na^+$, $BBN(OCHO)_2^-$], [$Cy_3PH^+$, $BBN(OCHO)_2^-$ ],[$TMPH^+$, $BBN(OCHO)_2^-$] sind.

**7.** Verfahren nach einem der Ansprüche 1 bis 6, **dadurch gekennzeichnet, dass** es in einem oder einem Gemisch von mindestens zwei Lösungsmitteln stattfindet, ausgewählt aus der Gruppe, die gebildet wird aus:

- den Ethern, ausgewählt aus dem diethylischen Ether, dem THF, dem Diglym, dem 1,4-Dioxan;
- den Kohlenwasserstoffen, ausgewählt aus dem Benzol oder dem Toluol;
- den stickstoffhaltigen Lösungsmitteln, ausgewählt aus dem Pyridin oder dem Acetonitril;
- den Sulfoxiden, ausgewählt aus dem Dimethylsulfoxid;
- den Alkylhalogeniden, ausgewählt aus dem Chloroform oder dem Methylchlorid; und
- einer überkritischen Flüssigkeit, ausgewählt aus dem überkritischen $CO_2$.

**8.** Verfahren nach einem der Ansprüche 1 bis 7, **dadurch gekennzeichnet, dass** die Menge der ungesättigten organischen Verbindungen, die zu reduzieren ist, in Bezug auf das Borformiat der Formel (I) 0,5 bis 2 Moläquivalente beträgt.

**9.** Verfahren nach einem der Ansprüche 1 bis 8, **dadurch gekennzeichnet, dass** es bei Vorhandensein eines Zusatzstoffes stattfindet, der ausgewählt wird aus:

- den Kronenethern, ausgewählt aus der Gruppe, die aus der 12-Krone-4, der 15-Krone-5, der 18-Krone-6, der Dibenzo-18-Krone-6, der Benzo-18-Krone-6, der Benzo-15-Krone-5 und der Dibenzo-15-Krone-5 gebildet wird,
- den Azakronen, ausgewählt aus der Gruppe, die aus dem 1,4,7,10-Tetraazacyclododecan (Cyclen), dem 1,4,7,10,13,16-Hexaazacyclooctadecan (Hexacyclen) und der Diaza-18-Krone-6 gebildet wird;
- den Kronenthioethern, ausgewählt aus der Gruppe, die aus dem 1,5,9,13-Tetrathiacyclohexadecan (16-Ane-$S_4$) und dem 1,4,7,10,13,16-Hexathiacyclooctadecan (18-Ane-$S_4$) gebildet wird.

**10.** Verfahren nach Anspruch 9, **dadurch gekennzeichnet, dass** die Menge des Zusatzstoffes, in Bezug auf das Borformiat der Formel (I), Grenzwerte eingeschlossen, 1 bis 2 Moläquivalente beträgt.

**11.** Verwendung eines Verfahrens zur Reduktion ungesättigter organischer Verbindungen, die aus der Gruppe ausgewählt werden, die aus den Aldehyden, den Ketonen, den Iminen, den Carbonsäuren, den Amiden und den Estern gebildet wird, mit den Borformiaten der Formel (I) nach einem der Ansprüche 1 bis 10,

- für die Vorbereitung von Methanol, von methylierten Aminen der Formel $R^1R^2N\text{-}CH_3$; von Formaldehyd und von Alkoholen der Formel $R^1CH_2OH$, wobei $R^1$ und $R^2$ wie in einem der Ansprüche 1, 3 und 4 definiert sind;
- für die Vorbereitung von Reagenzien für die Suzuki-Kupplungsreaktionen,
- bei der Herstellung von Vitaminen, von pharmazeutischen Erzeugnissen, von Klebstoffen, von Acrylfasern, von synthetischen Ledern, von Pestiziden.

**12.** Verfahren zur Vorbereitung von Methanol, umfassend

(i) einen Schritt einer Reduktion von Ameisensäure oder eines ihrer Ester der Formel $HCO_2R^8$, wobei $R^8$ aus einem Wasserstoffatom, einer Alkylgruppe oder einer Arylgruppe ausgewählt wird, mit einem Borformiat der Formel (I) nach dem Verfahren nach einem der Ansprüche 1 bis 10,
und gegebenenfalls
(ii) einen Schritt einer Hydrolyse.

**13.** Verfahren zur Vorbereitung von methylierten Aminen der Formel $R^1R^2N\text{-}CH_3$, wobei $R^1$ und $R^2$ wie in einem der Ansprüche 1, 3 und 4 definiert sind, umfassend

(i) einen Schritt einer Reduktion von Ameisensäure bei Vorhandensein eines primären Amins oder eines sekundären Amins, mit einem Borformiat der Formel (I) nach dem Verfahren nach einem der Ansprüche 1 bis 10, und
(ii) eine Destillation oder eine Vakuumkonzentration oder eine Säulenchromatographie.

**14.** Verfahren zur Vorbereitung von Formaldehyd, umfassend einen Schritt einer Reduktion von Ameisensäure oder eines ihrer Ester der Formel $HCO_2R^8$, wobei $R^8$ aus einem Wasserstoffatom, einer Alkylgruppe oder einer Arylgruppe ausgewählt wird, mit einem Borformiat der Formel (I) nach dem Verfahren nach einem der Ansprüche 1 bis 10,
und gegebenenfalls
(ii) einen Schritt einer Hydrolyse.

**15.** Verfahren zur Vorbereitung eines Alkohols der Formel $R^1CH_2OH$, wobei $R^1$ wie in einem der Ansprüche 1, 3 und 4 definiert ist, umfassend

(i) einen Schritt einer Reduktion einer ungesättigten organischen Verbindung, die aus der Gruppe ausgewählt wird, die aus den Aldehyden, den Carbonsäuren und den Estern gebildet wird, mit einem Borformiat der Formel (I) nach dem Verfahren nach einem der Ansprüche 1 bis 10, und
gegebenenfalls
(ii) einen Schritt einer Hydrolyse.

## Claims

**1.** Method for reducing unsaturated organic compounds selected from the group formed by aldehydes, ketones, imines, carboxylic acids, amides and esters, **characterised in that** said unsaturated organic compound is made to react with a boron formate of formula ((I)

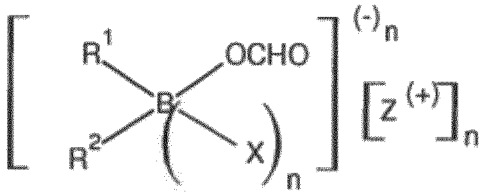

(I)

in which

- $R^1$ and $R^2$, independently of one another, are selected from a hydroxyl group, an alkoxy group, an alkyl group, an alkenyl group, an alkynyl group, an aryl group, a heteroaryl group, a heterocyclic group, a halogen atom, a silyl group, a siloxy group, a phosphino group, and an amino group, said alkyl, alkenyl, alkynyl, alkoxy, silyl, siloxy, aryl, phosphino and amino groups optionally being substituted; or
- $R^1$ and $R^2$ taken together with the boron atom to which they are bonded, form an optionally substituted heterocycle;
- X is selected from the group formed by a halogen atom, -OCHO and a sulfonate group of formula $-OSO_2R^7$, in which $R^7$ is selected from a methyl group ($CH_3$), a trifluoromethyl group ($CF_3$), a toluene group ($p$-$CH_3C_6H_4$) or a benzene group ($C_6H_5$),
- Z is a cation selected from the group formed by a protonated organic base having a pKa greater than 3.7 chosen from the group formed by triethylammonium ($HNEt_3^+$), di-isopropylethylammonium ($i$-$Pr_2EtNH^+$), 2,2,6,6-tetramethylpiperidinium ($TMPH^+$), and tricyclohexylphosphonium ($HPCy_3^+$); $Na^+$; $Li^+$; $K^+$; $Cs^+$; tetraphenylphosphonium ($PPh_4^+$); tetramethylammonium ($NMe4^+$); tetraethylammonium ($NEt_4^+$); tetrabutylammonium ($NBu_4^+$) and tetraphenylammonium ($NP_4^+$);
- n is an integer equal to 1;

in the presence of a solvent and optionally of an organic or inorganic base,
with the understanding that the boron formates of formula (I) in which n is equal to 1 are not frustrated Lewis pairs.

**2.** Method according to claim 1, **characterised in that**

- the aldehydes, the ketones, the carboxylic acids and the esters, are reduced to alcohols;
- the imines are reduced to amines; and
- the amides are reduced to amines or to alcohols.

**3.** Method according to one of claims 1 or 2, **characterised in that** in the boron formate of formula (I),

- $R^1$ and $R^2$, independently of one another, are selected from the group formed by an alkyl group comprising 1 to 12 carbon atoms; an aryl comprising 6 to 20 carbon atoms, said alkyl and aryl groups optionally being substituted.

4. Method according to one of claims 1 or 2, **characterised in that** in the boron formate of formula (I),

- $R^1$ and $R^2$ taken together with the boron atom to which they are bonded form a heterocycle comprising from 5 to 10 members, said heterocycle optionally being substituted.

5. Method according to any of claims 1 to 4, **characterised in that** Z is a cation selected from the group formed by triethylammonium ($HNEt_3^+$), di-isopropylethylammonium ($i$-$Pr_2EtNH^+$), 2,2,6,6-tetramethylpiperidinium ($TMPH^+$), tricyclohexylphosphonium ($HPCy_3^+$), and $Na^+$.

6. Method according to any of claims 1 to 5, **characterised in that** the boron formates of formula (I) are [$Et_3NH^+$, $BCy_2(OCHO)_2^-$], [i-$Pr_2EtNH^+$, $BCy_2(OCHO)_2^-$], [$Et_3NH^+$, n-$Bu_2B(OCHO)_2^-$], [$Et_3NH^+$, $BBN(OCHO)_2^-$], [i-$Pr_2EtNH^+$, $BBN(OCHO)_2^-$], [$Na^+$, $BBN(OCHO)_2^-$], [$Cy_3PH^+$, $BBN(OCHO)_2^-$] and [$TMPH^+$, $BBN(OCHO)_2^-$].

7. Method according to any of claims 1 to 6, **characterised in that** it takes place in one or a mixture of at least two solvents selected from the group formed by:

- the ethers selected from diethyl ether, THF, diglyme, 1,4-dioxane;
- the hydrocarbons selected from benzene, or toluene;
- the nitrogen solvents selected from pyridine, or acetonitrile;
- the sulfoxides selected from dimethylsulfoxide;
- the alkyl halides selected from chloroform, or methylene chloride; and
- a supercritical fluid selected from supercritical $CO_2$.

8. Method according to any of claims 1 to 7, **characterised in that** the quantity of unsaturated organic compounds to be reduced is from 0.5 to 2 molar equivalents, with respect to the boron formate of formula (I).

9. Method according to any of claims 1 to 8, **characterised in that** it takes place in the presence of an additive selected from:

- the crowns ethers selected from the group formed by 12-crown-4, 15-crown-5, 18-crown-6, dibenzo-18-crown-6, benzo-18-crown-6, benzo-15-crown-5, and dibenzo-15-crown-5;
- the aza-crowns selected from the group formed by 1,4,7,10-tetraazacyclododecane (cyclen), 1,4,7,10,13,16-hexaazacyclooctadecane (hexacyclen), and diaza-18-crown-6;
- the crown thioethers selected from the group formed by 1,5,9,13-tetrathiacyclohexadecane (16-Ane-$S_4$), and 1,4,7,10,13,16-hexathiacyclooctadecane (18-Ane-$S_6$).

10. Method according to claim 9, **characterised in that** the quantity of the additive is from 1 to 2 molar equivalents, limits included, with respect to the boron formate of formula (I).

11. Use of a method for reducing unsaturated organic compounds selected from the group formed by aldehydes, ketones, imines, carboxylic acids, amides and esters with the boron formates of formula (I) according to any of claims 1 to 10,

- for the preparation of methanol, methylated amines of formula $R^1R^2N$-$CH_3$, formaldehyde and alcohols of formula $R^1CH_2OH$ with $R^1$ and $R^2$ as defined in one of claims 1, 3 and 4;
- for the preparation of reagents for Suzuki coupling reactions,
- in the production of vitamins, pharmaceutical products, adhesives, acrylic fibres, synthetic leathers and pesticides.

12. Method for the preparation of methanol comprising

(i) a step of reducing formic acid or one of the esters thereof of formula $HCO_2R^8$ in which $R^8$ is selected from a hydrogen atom, an alkyl group, or an aryl group, with a boron formate of formula (I) according to the method of any of claims 1 to 10, and optionally
(ii) a step of hydrolysis.

13. Method for the preparation of methylated amines of formula $R^1R^2N$-$CH_3$ with $R^1$ and $R^2$ as defined in claims 1, 3 or 4, comprising

(i) a step of reducing formic acid in the presence of a primary amine or of a secondary amine, with a boron formate of formula (I) according to the method of any of claims 1 to 10, and
(ii) a distillation or a concentration under a vacuum or a column chromatography,

**14.** Method for the preparation of formaldehyde, comprising a step of reducing formic acid or one of the esters thereof of formula $HCO_2R^8$ in which $R^8$ is selected from a hydrogen atom, an alkyl group, or an aryl group, with a boron formate of formula (I) according to the method of any of claims 1 to 10, and optionally
(ii) a step of hydrolysis.

**15.** Method for the preparation of an alcohol of formula $R^1CH_2OH$ with $R^1$ as defined in claims 1, 3 or 4, comprising

(i) a step of reducing an unsaturated organic compound selected from the group formed by the aldehydes, the carboxylic acids and the esters with a boron formate of formula (I) according to the method of any of claims 1 to 10, and optionally
(ii) a step of hydrolysis.

Préparation de $B(OCH_3)_3$ à partir du borax naturel

$$Na_2B_4O_7 \cdot 10\,H_2O + 2\,HCl \longrightarrow 4\,B(OH)_3 + 2\,NaCl + 5\,H_2O$$

$$B(OH)_3 + 3\,CH_3OH \longrightarrow B(OCH_3)_3 + 3\,H_2O$$

Préparation de NaH

$$2\,Na + H_2 \longrightarrow 2\,NaH$$

---

Synthèse du TBHS

$$B(OCH_3)_3 + 4\,NaH \longrightarrow NaBH_4 + 3\,NaOCH_3$$

**Figure 1**

$$3\,HCO_2H_{(l)} \xrightarrow{\text{catalyseur}} CH_3OH_{(l)} + 2\,CO_{2(g)} + H_2O_{(l)} \quad (3)$$

(Equation 3)

**Figure 2**

$$HCO_2H_{(l)} \xrightarrow{\text{catalyseur}} CO_{2(g)} + H_{2(g)} \quad \text{(Equation 4)}$$

$$HCO_2H_{(l)} \xrightarrow{\text{catalyseur}} CO_{(g)} + H_2O_{(l)} \quad \text{(Equation 5)}$$

**Figure 3**

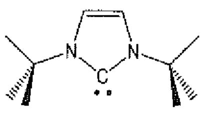
ItBu

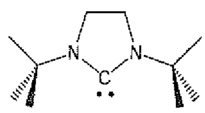
s-ItBu

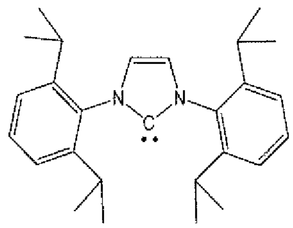
IPr

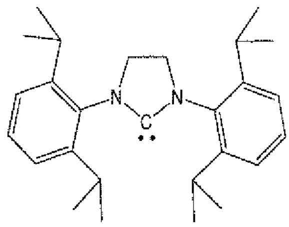
s-IPr

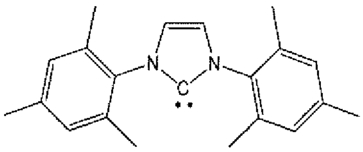
IMes

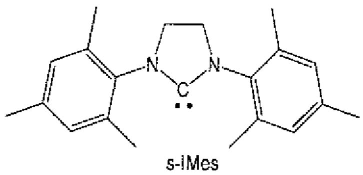
s-IMes

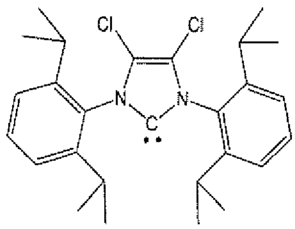
$Cl_2$-IPr

**Figure 4**

$$CO_2 + 2\,e^- + 2H^+ \text{ ou } CO_2 + H_2 \longrightarrow HCO_2H \text{ ou } HCO_2^-, \text{ Base } H^+$$

**Figure 5**

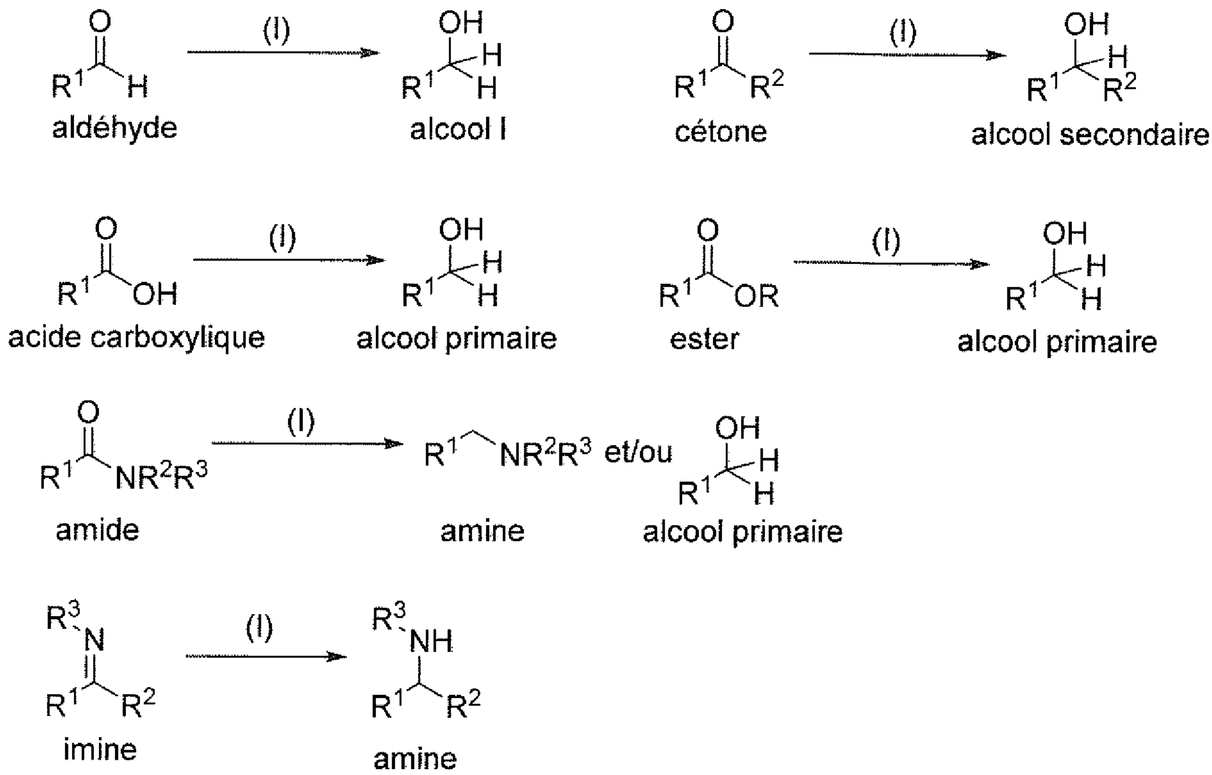
(I)
aldéhyde
alcool I
(I)
cétone
alcool secondaire
(I)
acide carboxylique
alcool primaire
(I)
ester
alcool primaire
(I)
et/ou
amide
amine
alcool primaire
(I)
imine
amine

**Figure 6**

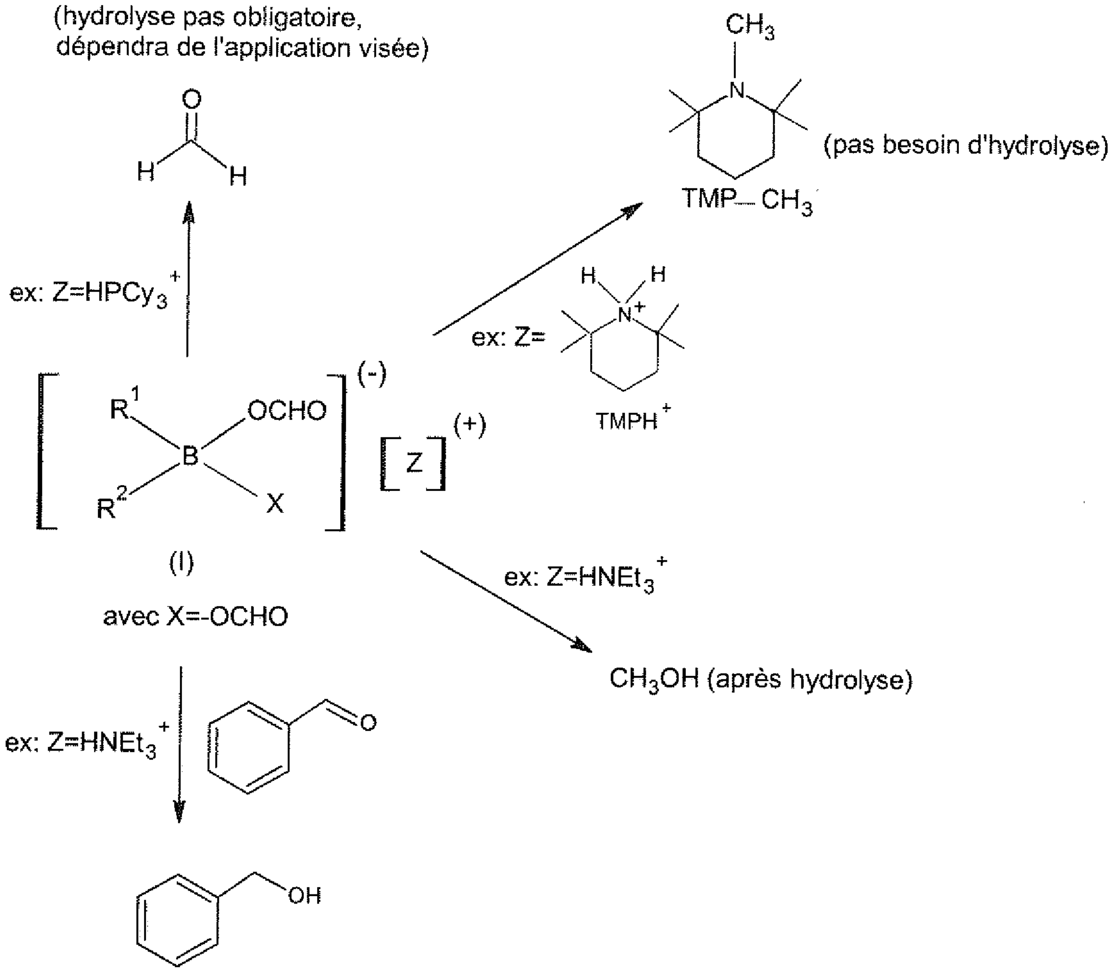


**Figure 7**

$$\underbrace{(C_6F_5)_3B + tBu_3P}_{\text{FLP}} \underset{}{\overset{\text{H–H}}{\rightleftharpoons}} [tBu_3PH^+,HB(C_6F_5)_3^-] \overset{CO_2}{\rightleftharpoons} [tBu_3PH^+,OHCOB(C_6F_5)_3^-]$$

**Figure 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2011045605 A1


### Littérature non-brevet citée dans la description


- *Organometallics,* 2013, vol. 32, 2459-2462
- **A. FUJII ; S. HASHIGUCHI ; N. UEMATSU ; T. IKARIYA ; R. NOYORI.** *J. Am. Chem. Soc.,* 1996, vol. 118, 2521-2522
- **G. BRIEGER ; T. J. NESTRICK.** *Chem. Rev.,* 1974, vol. 74, 567-580
- **D. WANG ; D. ASTRUC.** *Chem. Rev.,* 2015, vol. 115, 6621-6686
- **G. WIENHOFER ; I. SORRIBES ; A. BODDIEN ; F. WESTERHAUS ; K. JUNGE ; H. JUNGE ; R. LLUSAR ; M. BELLER.** *J. Am. Chem. Soc.,* 2011, vol. 133, 12875-12879
- **R. SHEN ; T. CHEN ; Y. ZHAO ; R. QIU ; Y. ZHOU ; S. YIN ; X. WANG ; M. GOTO ; L. B. HAN.** *J. Am. Chem. Soc.,* 2011, vol. 133, 17037-17044
- **P. HAUWERT ; G. MAESTRI ; J. W. SPRENGERS ; M. CATELLANI ; C. J. ELSEVIER.** *Angew. Chem. Int. Ed. Engl.,* 2008, vol. 47, 3223-3226
- **A. J. MILLER ; D. M. HEINEKEY ; J. M. MAYER ; K. I. GOLDBERG.** *Angew. Chem. Int. Ed. Engl.,* 2013, vol. 52, 3981-3984
- **S. SAVOUREY ; G. LEFEVRE ; J. C. BERTHET ; P. THUERY ; C. GENRE ; T. CANTAT.** *Angew. Chem. Int. Ed. Engl.,* 2014, vol. 53, 10466-10470
- **M. C. NEARY ; G. PARKIN.** *Chem. Sci.,* 2015, vol. 6, 1859-1865
- **S. SAVOUREY ; G. LEFEVRE ; J. C. BERTHET ; T. CANTAT.** *Chem. Commun.,* 2014, vol. 50, 14033-14036
- **ILONA PEUSER et al.** CO2 and Formate Complexes of Phosphine/Borane Frustrated Lewis Pairs. *CHEMISTRY - A EUROPEAN JOURNAL,* 22 Août 2011, vol. 17 (35), 9640-9650
- *DALTON TRANSACTIONS: THE INTERNATIONAL JOURNAL FOR INORGANIC, ORGANOMETALLIC AND BIOINORGANIC CHEMISTRY,* 2012, vol. 41 (30), 9061
- **R. TANAKA ; M. YAMASHITA ; K. NOZAKI.** *J. Am. Chem. Soc.,* 2009, vol. 131, 14168-14169
- **Y. HORI ; H. WAKEBE ; T. TSUKAMOTO ; O. KOGA.** *Electrochim. Acta,* 1994, vol. 39, 1833-1839
- **N. FURUYA ; T. YAMAZAKI ; M. SHIBATA.** *J. Electroanal. Chem.,* 1997, vol. 431, 39-41
- **A. GOEPPERT ; M. CZAUN ; J. P. JONES ; G. K. SURYA PRAKASH ; G. A. OLAH.** *Chem. Soc. Rev.,* 2014, vol. 43, 7995-8048
- **H.-R. M. JHONG ; S. MA ; P. J. A. KENIS.** *Curr. Opin. Chem. Eng.,* 2013, vol. 2, 191-199
- **X. LU ; D. Y. C. LEUNG ; H. WANG ; M. K. H. LEUNG ; J. XUAN.** *ChemElectroChem,* 2014, vol. 1, 836-849
- **A. ABIKO.** *Org. Synth.,* 2002, vol. 79, 103